# EUROPEAN PATENT APPLICATION

(11) **EP 4 847 611 A1**
(43) Date of publication of application: **02.12.2026**
(21) Application number: 24931124.2
(22) Date of filing: 04.12.2024
(51) Int. Cl.: C12N 9/90, C12N 9/12, C12N 15/86, C12N 15/67, A61K 48/00, A61P 21/00

(54) **AAV-BASED GENE THERAPEUTIC AGENT FOR TREATING GNE MYOPATHY**

(30) Priority: 20.03.2024 KR 20240038584; 15.11.2024 KR 20240163342
(71) Applicant: Kolon Industries, Inc., Seoul 07793 (KR)
(72) Inventor: SHIN, Seung Kyun, Seoul 07793 (KR); CHOI, Heon Sik, Seoul 07793 (KR); SHIN, Yu Bin, Seoul 07793 (KR)
(74) Representative: Vossius & Partner Patentanwälte Rechtsanwälte mbB
(86) International application number: PCT/KR2024/019754
(87) International publication number: WO 2025/198126

(57) **Abstract**

The present application relates to an adeno-associated virus (AAV)-based gene therapy for treating GNE myopathy (GNEM), which is a next-generation biopharmaceutical gene therapy technology. The present application provides an isolated nucleic acid having a sequence identity of 75 % to 99 % with a nucleotide sequence of SEQ ID NO: 2 and having a GC-content of 50 to 60 % with respect to the entire nucleotide sequence, an expression cassette including the isolated nucleic acid, an adeno-associated virus serotype 9-based recombinant virus including the expression cassette, and a pharmaceutical composition for preventing or treating GNEM, including the recombinant virus as an active ingredient.

## Description

### Technical Field

The present application relates to gene therapy technology, a next-generation biopharmaceutical, and more specifically, to an AAV-based gene therapy for treating GNE myopathy (GNEM). The present application claims the benefit of Korean Patent Application No. 10-2024-0038584, filed March 20, 2024, and Korean Patent Application No. 10-2024-0163342, filed November 15, 2024, the disclosures of which are incorporated herein by reference.

### Background Art

GNE myopathy (GNEM) is a hereditary muscle disease associated with mutations in the GNE gene. GNEM is a rare, currently incurable disease that is characterized by progressive muscle degeneration that typically begins in distal muscles and can extend throughout the body. In patients with GNEM, reduced GNE enzyme activity is commonly associated with decreased sialic acid biosynthesis, and this decrease is believed to be a major contributing factor to muscle loss. GNEM occurs on average in people in their 20s to 40s, and the initial symptoms are weakness in the tibialis anterior muscles, causing foot drop and making it difficult to lift the ankle, as well as toe dragging and an abnormal gait. As the disease progresses, postural stability may decrease, which can result in frequent falls and difficulty with activities such as running, climbing stairs, and rising from a seated position. In GNEM, muscle involvement may relatively spare the quadriceps femoris compared to other limb muscles however, upper limb muscles may also become affected, which can cause limitations in activities of daily living. GNEM progresses over decades, and cervical muscles may also be affected, leading to reduced mobility and, in advanced stages, severe impairment of skeletal muscle function in the upper and lower extremities.

The GNE protein is an enzyme protein called UDP-N-acetylglucosamine-2-epimerase/N-acetylmannosamine kinase, which is directly involved in the biosynthesis of sialic acid. The GNE protein consists of 753 amino acids and includes two functional domains, the glucosamine (UDP-N-acetyl)-2-epimerase domain (the region from the N-terminus to amino acid position 410) and the N-acetylmannosamine kinase domain (the region from amino acid position 411 to the C-terminus). Mutations in the GNE gene may occur at various positions within the DNA sequences encoding the two functional domains described above, and mutations that reduce GNE enzyme activity have been reported to cause GNEM symptoms by decreasing the level of sialylation of glycoproteins expressed on the muscle cell surface.

There is currently no cure for GNEM, and conservative treatment focuses on symptoms. Although periodic physical therapy and management after early diagnosis of GNEM may slow the progression of GNEM, it is not a method that may fundamentally treat muscle atrophy in patients caused by continuous muscle use, and has limitations in improving the quality of life of patients. Accordingly, methods for providing N-acetylmannosamine (ManNAc), an intermediate in the sialic acid biosynthetic pathway, and methods for inducing continuous expression of normal GNE genes by using gene therapy are being attempted, but are still inadequate.

Against this technical background, as a result of efforts to develop an adeno-associated virus (AAV) vector-based gene therapy for treating GNEM, a novel genetic construct with improved GNE expression efficiency was derived, and, based on this, the present disclosure was completed.

As a result, the isolated nucleic acid for treating GNEM, the expression cassette including the same, the recombinant virus including the same, or the pharmaceutical composition including the same, manufactured according to the present disclosure, may be utilized in the field of next-generation biopharmaceuticals, and may correspond to an innovative new drug as it is a gene therapy that has never been used before in relation to GNEM.

### Disclosure of Invention

### Technical Problem

One aspect is to provide an isolated nucleic acid engineered to enhance the level of expression of a GNE protein.

Another aspect is to provide an expression cassette including the isolated nucleic acid.

Another aspect is to provide an adeno-associated virus serotype 9 based recombinant virus including the expression cassette.

Another aspect is to provide a pharmaceutical composition for preventing or treating GNE myopathy (GNEM) including a vector including the isolated nucleic acid.

Other objectives and advantages of the present application will become apparent from the following detailed description, together with the appended claims and drawings. Any matter not described herein will be sufficiently recognized and inferred by a person of ordinary skill in the art of the present application or a similar art, and the description is hereby omitted.

### Solution to Problem

Each of the descriptions and embodiments disclosed in the present application may be applied to other descriptions and embodiments. Accordingly, all combinations of the various elements disclosed in the present application fall within the scope of the present application. Furthermore, the scope of the present application is not to be considered limited by the specific description set forth below.

One aspect is to provide an isolated nucleic acid having a sequence identity of 75 % to 99 % with the nucleotide sequence of SEQ ID NO: 2 and a GC-content of 50 to 60 % based on the entire nucleotide sequence.

As used herein, the term "nucleic acid" refers to a polymeric substance including a plurality of nucleotide units, specifically, a polymer in which a plurality of nucleotide units are linked to each other by phosphodiester bonds of a sugar/phosphate backbone. The nucleic acids may be used interchangeably with the terms "polynucleotide" and "nucleic acid molecule." The nucleic acid is a biopolymer essential to living organisms and may be RNA or DNA, preferably DNA, which encodes genetic information through a unique nucleic acid sequence. The nucleic acid is isolated, artificially synthesized, or non-naturally occurring or engineered, wherein "non-naturally occurring or engineered" refers to a state created by artificial modification rather than a state of existence that occurs in a natural state. Here, the artificial modification is intended to improve the expression of the GNE protein and may include a coding sequence of a codon optimized GNE protein.

As used herein, the term "isolated" nucleic acid (for example, "isolated DNA") refers to a polynucleotide that is at least partially separated from other components of a naturally occurring organism or virus, for example, cellular or viral structural components or other polypeptides or at least a portion of the nucleic acid that is normally found associated with the polynucleotide.

As used herein, the term "codon optimized" refers to a gene or coding region of a nucleic acid molecule for transformation of a variety of hosts, and refers to altering codons in the gene or coding region of a nucleic acid molecule to reflect the typical codon usage of the host organism without altering the polypeptide encoded by the DNA. Such optimization includes replacing at least one, or one or more, or a significant number of codons with one or more codons that are more frequently used in the genes of that organism.

As used herein, the term "GNE (glucosamine (UDP-N-acetyl)-2-epimerase/*N-*acetylmannosamine kinase) protein" is an enzyme involved in the biosynthesis of sialic acid, which is responsible for two enzymatic reactions, from UDP-GIcNAc to ManNAc and from ManNAc to ManNAc6-phosphate. Accordingly, mutations in the GNE gene result in decreased sialic acid production, leading to decreased sialylation of various glycoproteins, including important muscle proteins such as alpha-dystroglycan (α-DG), neural cell adhesion molecule (NCAM) or neprilysin, or to decreased expression of other genes such as ganglioside (for example: GM3) synthetase, resulting in muscle degenerative changes.

In an embodiment, the GNE protein may consist of an amino acid sequence of SEQ ID NO: 1, and the wild-type GNE gene encoding the GNE protein may consist of a nucleotide sequence of SEQ ID NO: 2. The isolated nucleic acid according to an aspect may be intended to enhance expression of a GNE protein, for example, to enhance expression of a target protein under an AAV9 based expression construct, and may have 75 % to 99 % sequence identity to the nucleotide sequence of SEQ ID NO: 2 and a GC-content that corresponds to a range of 50 to 60 % based on the entire nucleotide sequence.

As used herein, the term "identity" refers to the overall relatedness between polymer molecules, for example, between nucleic acids (for example, DNA molecules and/or RNA molecules) and/or between polypeptides. For example, polypeptides are considered "substantially identical" to each other if their amino acid sequences are at least, 60 %, 65 %, 70 %, 75 %, 80 %, 85 %, 90 %, 95 %, or 99 % identical. Calculation of the percentage identity of two nucleic acid or polypeptide sequences may be performed, for example, by aligning the two sequences for optimal comparison purposes (for example, gaps may be introduced in one or both of the first and second sequences for optimal alignment, and non-identical sequences may be ignored for comparison purposes). For example, the length of the aligned sequence for comparison purposes is at least 60 %, at least 70 %, at least 80 %, at least 90 %, at least 95 %, or substantially 100 % of the length of the reference sequence. Next, the nucleic acid or polypeptide sequences at the corresponding positions are compared. Determination of percent identity between two sequences and comparison of sequences may be accomplished using mathematical algorithms. As is well known to a person of ordinary skill in the art, amino acid or nucleic acid sequences may be compared using any of a variety of algorithms, including those available in commercial computer programs such as BLASTN for nucleotide sequences and BLASTP, gapped BLAST, and PSIBLAST for amino acid sequences.

In an embodiment, the isolated nucleic acid may have from 75 % to 99 % sequence identity to the nucleotide sequence of SEQ ID NO: 2, for example, the isolated nucleic acid may have from 75 to 99 %, 75 to 95 %, 75 to 90 %, 75 to 85 %, 75 to 80 %, 80 to 99 %, 80 to 95 %, 80 to 90 %, 80 to 85 %, 75 to 80 %, 85 to 99 %, 85 to 95 %, 85 to 90 %, or 90 to 95 % sequence identity to the nucleotide sequence of SEQ ID NO: 2.

As used herein, the term "GC-content" refers to the ratio of guanine (G) to cytosine (C) among the total bases of the target region, and it is known that the higher the GC-content, the higher the DNA density and the less likely it is to be denatured.

In an embodiment, the isolated nucleic acid may have a GC-content corresponding to the range of 50 to 60 % based on the entire nucleotide sequence, for example, the GC-content of the isolated nucleic acid may be 50 to 60 %, 50 to 58 %, 50 to 56 %, 50 to 54 %, 50 to 52 %, 52 to 60 %, 52 to 58 %, 52 to 56 %, 52 to 54 %, 54 to 60 %, 54 to 58 %, 54 to 56 %, 56 to 60 %, 56 to 58 %, or 58 to 60 %.

In an embodiment, the isolated nucleic acid may satisfy the following conditions: (a) encoding a GNE protein represented by the amino acid sequence of SEQ ID NO: 1; (b) sequence identity of 75 to 80 % with the nucleotide sequence of SEQ ID NO: 2; and (c) a GC-content of 55 to 58 % based on the entire nucleotide sequence. Specifically, the isolated nucleic acid may be any one selected from the group consisting of a nucleotide sequence of SEQ ID NO: 3, a nucleotide sequence of SEQ ID NO: 5, and a nucleotide sequence of SEQ ID NO: 6.

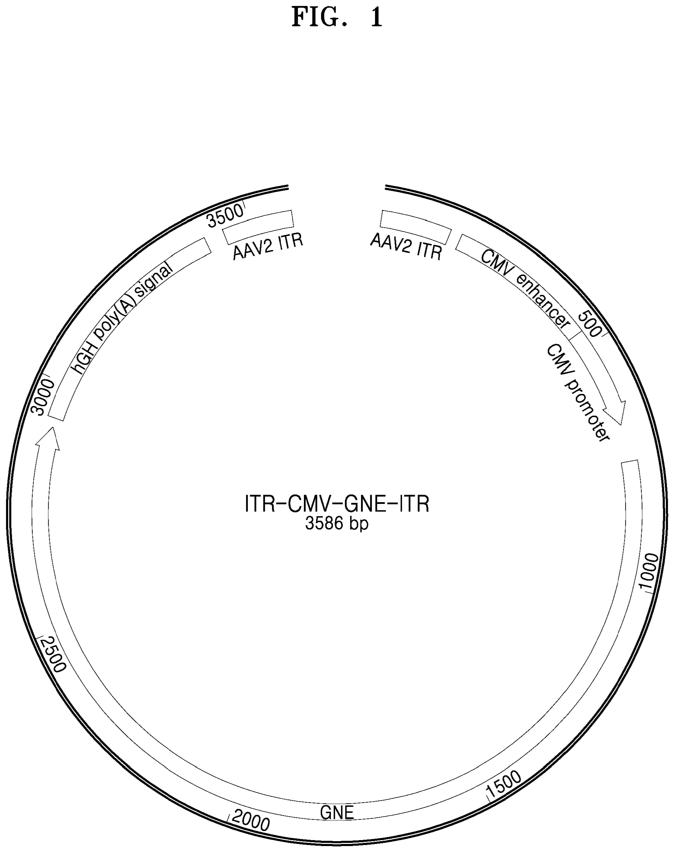

According to an embodiment, the isolated nucleic acid of the present disclosure is modified based on the wild-type GNE nucleotide sequence, and when the isolated nucleic acid is applied as a transgene (transplanted gene) in an expression cassette, as illustrated in FIG. 1, the level of expression of the GNE protein may be significantly enhanced.

Another aspect is to provide an expression cassette including the isolated nucleic acid.

Since the expression cassette includes or utilizes the isolated nucleic acid described above, the common contents between them are omitted to avoid excessive complexity of the present specification.

As used herein, the term "expression cassette" refers to a nucleic acid construct including elements necessary for expressing a target protein. Specifically, it refers to a nucleic acid construct including an expression control sequence including at least a promoter, and a nucleic acid for expressing a target protein operably linked to the expression control sequence.

As used herein, the term "expression control sequence" refers to a nucleic acid sequence that is essential for expressing a coding sequence for a target protein to which it is operably linked in a particular host organism. For example, it is known to use regulatory sequences suitable for eukaryotic cells, such as promoters, polyadenylation signals, and enhancers.

As used herein, the term "promoter" refers to a nucleic acid fragment that controls the transcription of one or more coding sequences and is located upstream in the reading direction from the transcription initiation site of the coding sequence, with respect to the direction of transcription, which is structurally identified by the presence of a binding site for DNA-dependent RNA polymerase, a transcription initiation site, and any other nucleic acid sequences, for example, without limitation, transcription factor binding sites, repressor and activator protein binding sites, and any other nucleotide sequence known to a person of ordinary skill in the art that directly or indirectly regulates the level of transcription of the promoter. Any promoter that functions in mammalian cells is exemplified as the promoter, and examples thereof are constitutive expression promoters such as the CMV promoter, the SV40 promoter, the EF-1α promoter, the CAG promoter, the PGK promoter, the U3 promoter, the U6 promoter, the H1 promoter, and the like. In addition to these promoters, known inducible promoters, tissue · organ-specific promoters, period-specific promoters, or mutant sequences having functional equivalents thereto may be used in the present disclosure.

As used herein, the term "enhancer" may refer to a nucleic acid sequence located adjacent to a sequence encoding a target protein. Enhancer elements are typically located in the 5' direction from promoter elements, or may be located downstream of, or within, the coding nucleic acid sequence (for example, a DNA sequence that is transcribed or translated into the recombinant product or products). That is, the enhancer element may be located 100 base pairs (bp), 200 bp, or 300 bp or more upstream or downstream from the nucleic acid sequence encoding the target protein.

In an embodiment, the expression cassette is intended to enhance expression of a target protein under an AAV9 based expression construct, and may include, or may be operably linked to, the following elements: a first inverted terminal repeat (ITR); a cytomegalovirus enhancer; a cytomegalovirus promoter; an isolated nucleic acid having at least 75 % to 99 % sequence identity to the nucleotide sequence of SEQ ID NO: 2 and having a GC-content corresponding to a range of 50 to 60 % based on the entire nucleotide sequence; a human growth hormone gene 1 (hGH1) polyadenylation signal ; and a second inverted terminal repeat (ITR).

As used herein, the term "operably linked" may refer to nucleotide sequences being linked on a single nucleic acid fragment such that one function is affected by the other.

Another aspect is to provide an adeno-associated virus serotype 9 (AAV9) based recombinant virus including the expression cassette.

Since the adeno-associated virus serotype 9 based recombinant virus includes or utilizes the isolated nucleic acid or expression cassette described above, the common contents therebetween are omitted to avoid excessive complexity of the present specification.

As used herein, the term "adeno-associated virus (AAV)" refers to a small (26 nm), non-enveloped parvovirus having a single-stranded genome of approximately 4.7 kb, which has been highlighted as a gene delivery vector for disease treatment due to its high transduction efficiency, sustained transgene expression, and low pathogenicity after infection. However, since adeno-associated virus is also a viral vector, like other viral vectors, it has the problem that high-dose systemic administration may cause serious side effects. The serotype of the adeno-associated virus is mainly determined by the structure of an AAV capsid protein, and since different capsid protein structures recognize different cell surface receptors, the selection of the serotype may affect the infection efficiency, tissue tropism, and expression initiation time of AAV. The serotypes of the adeno-associated viruses include, for example, AAV2, AAV3, AAV4, AAV5, AAV6, AAV7 AAV8, AAV9, AAV10, AAV11, AAV12, and others.

As used herein, the term "adeno-associated virus serotype 9 based recombinant virus" refers to the above-described expression cassette enclosed within an AAV capsid, and may be used interchangeably with the terms "AAV9-based virus-like particle," "AAV9 recombinant virus strain," "AAV9 recombinant vector," or "rAAV9 vector."

According to an embodiment, the recombinant virus of the present disclosure is introduced with an isolated nucleic acid modified based on the wild-type GNE nucleotide sequence, or an expression cassette including the nucleic acid. The recombinant virus may be introduced into a cell or administered in vivo to enhance expression efficiency of the GNE protein, thereby enhancing sialic acid production in muscle. In addition, the recombinant virus may significantly enhance the expression level of the GNE protein, thereby contributing to reducing side effects resulting from the application of the viral vector by reducing the effective dosage for treating the target disease.

Another aspect is to provide a pharmaceutical composition for the prevention or treatment of GNE myopathy (GNEM), including as an active ingredient a vector including any one isolated nucleic acid selected from the group consisting of the nucleotide sequence of SEQ ID NO: 3, the nucleotide sequence of SEQ ID NO: 5, and the nucleotide sequence of SEQ ID NO: 6, and a pharmaceutical use of the pharmaceutical composition for the prevention or treatment of GNEM.

Since the pharmaceutical composition for preventing or treating the GNEM includes or utilizes the isolated nucleic acid or expression cassette described above, the common contents therebetween are omitted to avoid excessive complexity of the present specification.

As used herein, the term "active ingredient" refers to an ingredient in an adequate effective amount that affects a beneficial or desirable clinical or biochemical outcome. Specifically, it may refer to an effective amount of a formulation, an active agent, or a recombinant virus. The effective amount may be administered once or more than once, and may be an adequate amount for preventing a disease or, without limitation, alleviating symptoms, reducing the extent of the disease, stabilizing (in other words, not worsening) the disease state, delaying or reducing the rate of disease progression, or improving or temporarily alleviating and alleviating (partially or completely) the disease state.

As used herein, the term "prevention" refers to any action that prevents the occurrence of a disease in advance, suppresses a disease, or delays its progression. For example, it refers to preventing the occurrence of obesity or characteristic features thereof, interfering with the occurrence thereof, or defending or protecting against the occurrence of GNEM or characteristic features thereof.

As used herein, the term "treatment" refers to both therapeutic treatment and prophylactic or preventative methods. It also refers to any action that improves or beneficially changes the symptoms of a disease. For example, preventing, reducing or improving the GNEM or a characteristic feature thereof, or delaying (attenuating) the progression of the disease or a characteristic feature thereof in a subject.

As used herein, the term "effective amount" refers to the meaning generally accepted in the art. The term may generally refer to an amount of a molecule, compound, or composition that will elicit an intended biological response (for example: a beneficial response) in a cell, tissue, system, animal, or human, as sought by a researcher, veterinarian, physician, or other clinician. Specifically, a "therapeutically effective amount" may refer to an amount of a molecule, compound, or composition that elicits a desirable medical response, such as a therapeutically relevant change in a measurable parameter associated with a disease or disorder, such that a particular clinical treatment may be considered effective. A therapeutically effective amount of a drug for treating the disease or disorder may be an amount necessary to cause a therapeutically relevant change in the parameter.

"GNE myopathy," a disease to be prevented or treated by the pharmaceutical composition above, is an autosomal recessive disease caused by a mutation in the GNE gene that produces an enzyme known as UDP-GIcNAc 2-Epimerase/ManNAc Kinase (GNE), and is also known by the terms "distal myopathy"," "Nonaka myopathy, vacuolar myopathy sparing the quadriceps femoris, inclusion body myopathy type 2 (IBM2 or HIBM2), or GNEM. Currently, there is no cure for GNEM, and treatment focuses on supportive care for symptoms, therefore, a multifaceted approach is required for the treatment of the disease.

In an embodiment, the present disclosure relates to a pharmaceutical composition including an AAV9-based recombinant virus including the isolated nucleic acid of the present disclosure in a pharmaceutically acceptable carrier or other pharmaceutical agent, adjuvant, diluent, or the like. For injection or systemic administration, the carrier will typically be a liquid carrier. For other administration methods, the carrier may be a solid or a liquid, for example, sterile pyrogen-free water or a sterile pyrogen-free phosphate-buffered saline solution. As an injection medium, it is preferable to use water containing additives common to injection solutions, such as stabilizers, salts or saline and/or buffers.

The pharmaceutical composition refers to a composition including the AAV9-based recombinant virus of the present disclosure and one or more component selected from the group consisting of pharmaceutically acceptable and pharmacologically appropriate excipients, such as fillers, solvents, diluents, carriers, adjuvants, disintegrants, delivery agents, preservatives, stabilizers, emulsifiers, suspending agents, thickeners, and long-term delivery regulators, wherein the selection and ratio thereof are determined according to the type, route, and dosage of administration. The pharmaceutical composition of the present disclosure and the method of preparing the same will be without a doubt obvious to those skilled in the art. Pharmaceutical compositions should preferably be manufactured in compliance with Good Manufacturing Practice (GMP) requirements. The composition may also include a buffer composition, a tonicity agent, a stabilizer and a solubilizer.

The pharmaceutical composition may be manufactured, packaged or sold broadly in the form of a single unit dose or multiple single unit doses in the form of a ready formulation. As used herein, the term "single unit dose" refers to discrete amount of a pharmaceutical composition containing a pre-specified amount of an active ingredient. The amount of active ingredient typically corresponds to a dosage of the active ingredient to be administered to the subject, or a convenient portion of such a dosage, for example, one-half or one-third of the dosage.

One aspect provides a method of preventing or treating GNEM including administering to a subject the pharmaceutical composition.

Since the method of preventing or treating the GNEM includes or utilizes the pharmaceutical composition described above, the common contents therebetween are omitted to avoid excessive complexity of the present specification.

As used herein, the term "subject" refers to a target in need of treatment of a disease, specifically GNEM, and more specifically may include all mammals such as a human or non-human primate, mouse, dog, cat, horse, cow, sheep, pig, goat, camel, or antelope.

### Advantageous Effects of Invention

According to an isolated nucleic acid of an aspect, and an expression cassette or an AAV9 based recombinant virus including the isolated nucleic acid, high-level expression of an GNE protein may be induced in a subject to which it is administered or introduced.

In addition, the AAV9 based recombinant virus according to an aspect may reduce the effective dosage as an effect resulting from the excellent GNE protein expression efficiency, and thus, may alleviate the side effects resulting from the application of the viral vector, thereby contributing to improving the efficacy of gene therapy for treating GNE myopathy (GNEM).

### Brief Description of Drawings

FIG. 1 is a diagram illustrating a structure of an expression cassette including a wild-type GNE nucleotide sequence (GNE wt) according to an embodiment.
FIG. 2 is a diagram illustrating a structure of an expression cassette including a GNE nucleotide sequence (GNE mod1) according to an embodiment.
FIG. 3 is a diagram illustrating a structure of an expression cassette including a GNE nucleotide sequence (GNE mod2) according to an embodiment.
FIG. 4 is a diagram illustrating a structure of an expression cassette including a GNE nucleotide sequence (GNE mod3) according to an embodiment.
FIG. 5 is a diagram illustrating a structure of an expression cassette including a GNE nucleotide sequence (GNE mod4) according to an embodiment.
FIG. 6 shows the results of confirming the expression level of a GNE protein by western blotting after transfecting HEK293T cells with a recombinant plasmid vector according to an embodiment.
FIG. 7 shows the results of confirming the expression level of a GNE protein by western blotting after transfecting C2C12 cells with a recombinant plasmid vector according to an embodiment.
FIG. 8 shows the results of confirming the expression level of a GNE protein by western blotting after transducing HEK293T cells with a recombinant AAV9 vector according to an embodiment.
FIG. 9 shows the results of confirming the expression level of GNE protein by western blotting after transducing C2C12 cells with a recombinant AAV9 vector according to an embodiment.
FIG. 10 shows the results of confirming the recovery of GNE protein expression by western blotting after transducing a recombinant AAV9 vector according to an embodiment into an mGne knockdown C2C12 cell line.
FIG. 11 shows the results of confirming the recovery of sialic acid levels after transducing a recombinant AAV9 vector according to an embodiment into an mGne knockdown C2C12 cell line, wherein FIG. 11A shows the results of flow cytometry of Neu5AC-expressing cells (SNA-FITC), and FIG. 11B shows the results of quantitatively comparing the results of the flow cytometry.
FIG. 12 shows the results of confirming the recovery of sialic acid levels after transducing a recombinant AAV9 vector according to an embodiment into an mGne knockdown C2C12 cell line, wherein FIG. 12A shows the results of flow cytometry of Galactose-expressing cells (PNA-FITC), and FIG. 12B shows the results of quantitatively comparing the results of the flow cytometry.
FIG. 13 shows the results of confirming the expression level of Neu5AC in muscle tissue through SNA staining in a mouse model administered with different dosages of a recombinant AAV9 vector including a GNE nucleotide sequence.
FIG. 14 shows the results of confirming the expression level of Galactose in muscle tissue through PNA staining in a mouse model administered with different dosages of a recombinant AAV9 vector including a GNE nucleotide sequence.
FIG. 15 shows the results of re-confirming the expression level of Neu5AC in muscle tissue through SNA staining in a mouse model administered with different dosages of a recombinant AAV9 vector including a GNE nucleotide sequence.
FIG. 16 shows the results of re-confirming the expression level of Galactose in muscle tissue through PNA staining in a mouse model administered with different dosages of a recombinant AAV9 vector including a GNE nucleotide sequence.
FIG. 17 shows the results of confirming the expression level of GNE mRNA in muscle tissue through qRT-PCR in groups of normal mice (WT) and disease mouse models administered with Mock AAV (Gne Kl Mock), according to an embodiment, wherein FIG. 17A shows the result of comparing the expression levels of the entire experimental group and the control group, and FIG. 17B shows the result of comparing the expression levels of the control group and the negative control group.
FIG. 18 shows the results of confirming the expression level of a GNE protein in muscle tissue by western blotting (detection antibody: #NBP1-81621) in a mouse model administered with different doses of a recombinant AAV9 vector including a GNE nucleotide sequence.
FIG. 19 shows the results of confirming the expression level of a GNE protein in muscle tissue by western blotting (detection antibody: #25079-1-ap) in a mouse model administered at different doses of a recombinant AAV9 vector including a GNE nucleotide sequence.
FIG. 20 shows the results of a treadmill test conducted on a mouse model administered with a recombinant AAV9 vector including a GNE nucleotide sequence according to an embodiment.
FIG. 21 shows the results of a treadmill test conducted on a mouse model administered with a recombinant AAV9 vector including a GNE nucleotide sequence according to an embodiment, comparing the results before and after administration.
FIG. 22 shows the results of a grip strength test conducted on a mouse model administered with a recombinant AAV9 vector including a GNE nucleotide sequence according to an embodiment.
FIG. 23 shows the results of a grip strength test conducted on a mouse model administered with a recombinant AAV9 vector including a GNE nucleotide sequence according to an embodiment, comparing the results before and after administration.
FIG. 24 shows the results of confirming the expression level of Neu5AC in muscle tissue by SNA staining in a mouse model administered with a recombinant AAV9 vector including a GNE nucleotide sequence according to an embodiment.
FIG. 25 shows the results of confirming the expression level of Galactose in muscle tissue by PNA staining in a mouse model administered with a recombinant AAV9 vector including a GNE nucleotide sequence according to an embodiment.
FIG. 26 shows the results of confirming the expression level of a GNE protein in muscle tissue by western blotting (detection antibody: #25079-1-ap) in a mouse model administered with a recombinant AAV9 vector including a GNE nucleotide sequence according to an embodiment.
FIG. 27 shows the results of comparing the expression level of a GNE protein in muscle tissue in a mouse model administered with a recombinant AAV9 vector including a GNE nucleotide sequence according to an embodiment.
FIG. 28 shows the results of confirming the change in body weight for 8 weeks after administration to a mouse model administered with a recombinant AAV9 vector including a GNE nucleotide sequence according to an embodiment.
FIG. 29 shows the results of a treadmill test conducted on a mouse model administered with a recombinant AAV9 vector including a GNE nucleotide sequence of Example 3.
FIG. 30 shows the results of confirming changes in body weight over 4 weeks after administration of a recombinant AAV9 vector including a GNE nucleotide sequence of Example 3 to a mouse model.

### Mode for the Invention

Hereinafter, preferred examples are presented to aid in the understanding of the present disclosure. However, the following examples are provided only to aid understanding of the present disclosure, and the present disclosure is not limited by the following examples.

### Experimental Example 1. Construction and Comparison of Expression Cassette for Expression of GNE Protein

### 1-1. Construction of Expression Cassette Including codon optimized Nucleotide Sequence

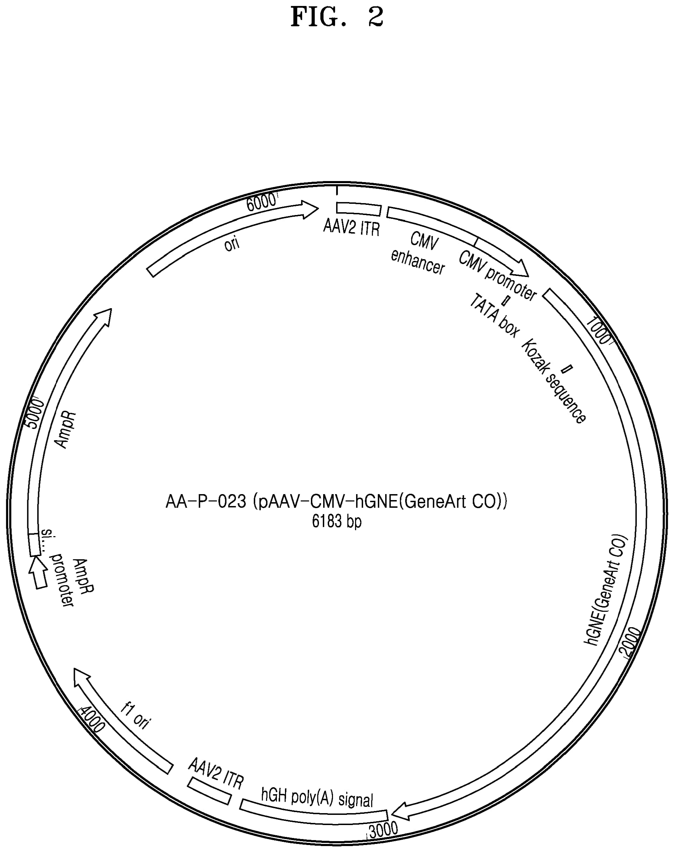

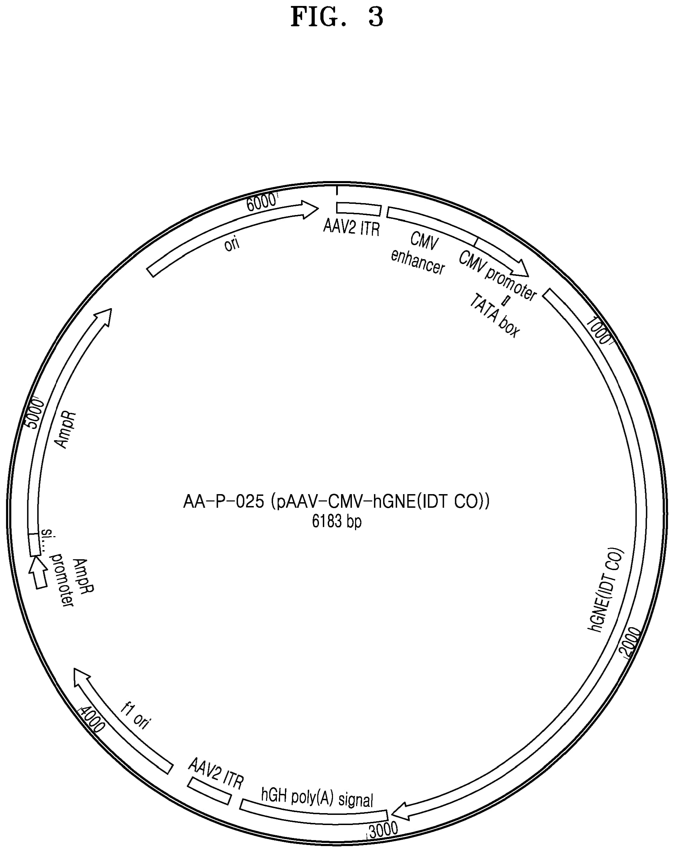

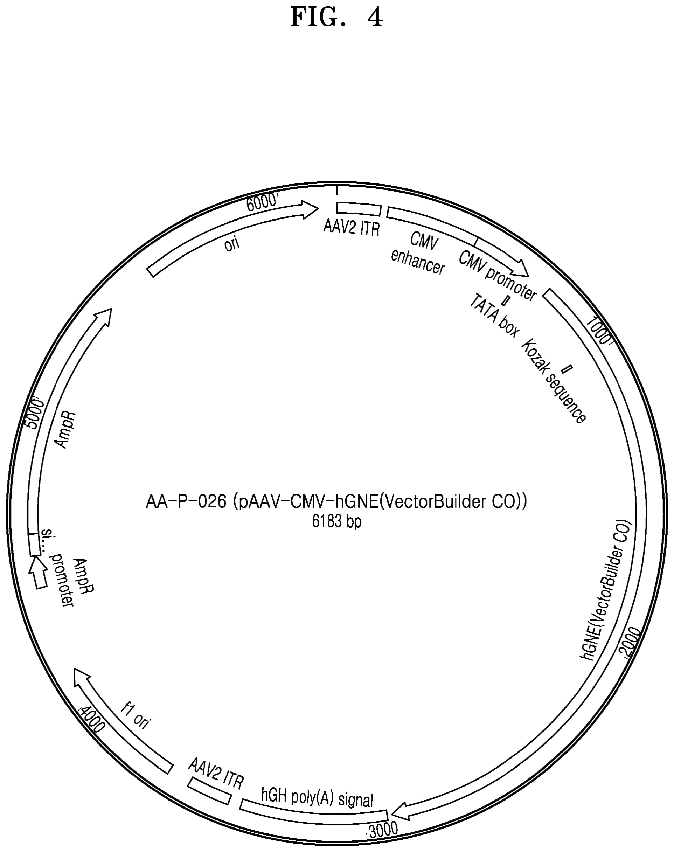

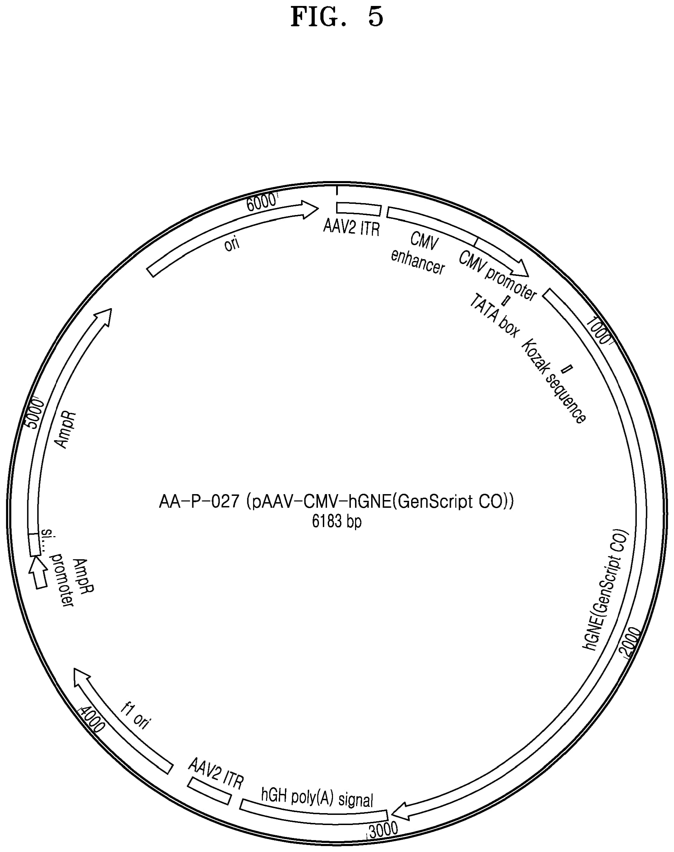

In the present experimental example, an expression cassette for the expression of GNE protein was prepared. To this end, as illustrated in FIG. 1 to FIG. 5, each expression cassette was prepared by modifying the nucleotide sequence of the GNE gene in a backbone including, from the 5' end to the 3' end, an AAV2 5' inverted terminal repeat (ITR), a cytomegalovirus (CMV) enhancer, a CMV promoter, a GNE gene (Transgene), a human growth hormone 1 (hGH1) polyadenylation signal, and an AAV2 3' ITR. As the GNE gene, nucleotides encoding the GNE protein consisting of the amino acid sequence of SEQ ID NO: 1 were applied, including the wild-type GNE nucleotide sequence (transcript variant 2, NM\_005476.7, 2169 bp) and a total of four codon optimized GNE nucleotide sequences (GNE mod1, GNE mod2, GNE mod3, GNE mod4). In the present experimental example, information on each GNE nucleotide sequence is as shown in Table 1 below.

**[Table 1]**

| Name | Sequence Information (from N-terminus to C-terminus or 5' to 3' end) | SEQ ID NO |
|---|---|---|
| GNE (wt, mod1~4) Amino acid | MEKNGNNRKLRVCVATCNRADYSKLAPIMFGIKTEPEFFELDVVVLGSHLI DDYGNTYRMIEQDDFDINTRLHTIVRGEDEAAMVESVGLALVKLPDVLNRL KPDIMIVHGDRFDALALATSAALMNIRILHIEGGEVSGTIDDSIRHAITKLAHY HVCCTRSAEQHLISMCEDHDRILLAGCPSYDKLLSAKNKDYMSIIRMWLG DDVKSKDYIVALQHPVTTDIKHSIKMFELTLDALISFNKRTLVLFPNIDAGSK EMVRVMRKKGIEHHPNFRAVKHVPFDQFIQLVAHAGCMIGNSSCGVREV GAFGTPVINLGTRQIGRETGENVLHVRDADTQDKILQALHLQFGKQYPCS KIYGDGNAVPRILKFLKSIDLQEPLQKKFCFPPVKENISQDIDHILETLSALA VDLGGTNLRVAIVSMKGEIVKKYTQFNPKTYEERINLILQMCVEAAAEAVKL NCRILGVGISTGGRVNPREGIVLHSTKLIQEWNSVDLRTPLSDTLHLPVWV DNDGNCAALAERKFGQGKGLENFVTLITGTGIGGGIIHQHELIHGSSFCAA ELGHLVVSLDGPDCSCGSHGCIEAYASGMALQREAKKLHDEDLLLVEGM SVPKDEAVGALHLIQAAKLGNAKAQSILRTAGTALGLGVVNILHTMNPSLVI | 1 |
| | LSGVLASHYIHIVKDVIRQQALSSVQDVDVVVSDLVDPALLGAASMVLDYT TRRIY | |
| GNE wt | ATGGAGAAGAATGGAAATAACCGAAAGCTGCGGGTTTGTGTTGCTACT TGTAACCGTGCAGATTATTCTAAACTTGCCCCGATCATGTTTGGCATTA AAACCGAACCTGAGTTCTTTGAACTTGATGTTGTGGTACTTGGCTCTCA CCTGATAGATGACTATGGAAATACATATCGAATGATTGAACAAGATGAC TTTGACATTAACACCAGGCTACACACAATTGTGAGGGGAGAAGATGAG GCAGCCATGGTGGAGTCAGTAGGCCTGGCCCTAGTGAAGCTGCCAGA TGTCCTTAATCGCCTGAAGCCTGATATCATGATTGTTCATGGAGACAG GTTTGATGCCCTGGCTCTGGCCACATCTGCTGCCTTGATGAACATCCG AATCCTTCACATTGAAGGTGGGGAAGTCAGTGGGACCATTGATGACTC TATCAGACATGCCATAACAAAACTGGCTCATTATCATGTGTGCTGCACC CGCAGTGCAGAGCAGCACCTGATATCCATGTGTGAGGACCATGATCG CATCCTTTTGGCAGGCTGCCCTTCCTATGACAAACTTCTCTCAGCCAA GAACAAAGACTACATGAGCATCATTCGCATGTGGCTAGGTGATGATGT AAAATCTAAAGATTACATTGTTGCACTACAGCACCCTGTGACCACTGAC ATTAAGCATTCCATAAAAATGTTTGAATTAACATTGGATGCACTTATCTC ATTTAACAAGCGGACCCTAGTCCTGTTTCCAAATATTGACGCAGGGAG CAAAGAGATGGTTCGAGTGATGCGGAAGAAGGGCATTGAGCATCATC CCAACTTTCGTGCAGTTAAACACGTCCCATTTGACCAGTTTATACAGTT GGTTGCCCATGCTGGCTGTATGATTGGGAACAGCAGCTGTGGGGTTC GAGAAGTTGGAGCTTTTGGAACACCTGTGATCAACCTGGGAACACGTC AGATTGGAAGAGAAACAGGGGAGAATGTTCTTCATGTCCGGGATGCTG ACACCCAAGACAAAATATTGCAAGCACTGCACCTTCAGTTTGGTAAACA GTACCCTTGTTCAAAGATATATGGGGATGGAAATGCTGTTCCAAGGAT TTTGAAGTTTCTCAAATCTATCGATCTTCAAGAGCCACTGCAAAAGAAA TTCTGCTTTCCTCCTGTGAAGGAGAATATCTCTCAAGATATTGACCATA TTCTTGAAACTCTAAGTGCCTTGGCCGTTGATCTTGGCGGGACGAACC TCCGAGTTGCAATAGTCAGCATGAAGGGTGAAATAGTTAAGAAGTATA CTCAGTTCAATCCTAAAACCTATGAAGAGAGGATTAATTTAATCCTACA GATGTGTGTGGAAGCTGCAGCAGAAGCTGTAAAACTGAACTGCAGAAT TTTGGGAGTAGGCATTTCCACAGGTGGCCGTGTAAATCCTCGGGAAG GAATTGTGCTGCATTCAACCAAACTGATCCAAGAGTGGAACTCTGTGG ACCTTAGGACCCCCCTTTCTGACACTTTGCATCTCCCTGTGTGGGTAG ACAATGATGGCAACTGTGCTGCCCTGGCGGAAAGGAAATTTGGCCAA GGAAAGGGACTGGAAAACTTTGTTACACTTATCACAGGCACAGGAATC GGTGGTGGAATTATCCATCAGCATGAATTGATCCACGGAAGCTCCTTC TGTGCTGCAGAACTGGGCCACCTTGTTGTGTCTCTGGATGGGCCTGAT TGTTCCTGTGGAAGCCATGGGTGCATTGAAGCATACGCCTCTGGAATG GCCTTGCAGAGGGAGGCAAAAAAGCTCCATGATGAGGACCTGCTCTT GGTGGAAGGGATGTCAGTGCCAAAAGATGAGGCTGTGGGTGCGCTCC ATCTCATCCAAGCTGCGAAACTTGGCAATGCGAAGGCCCAGAGCATCC TAAGAACAGCTGGAACAGCTTTGGGTCTTGGGGTTGTGAACATCCTCC ATACCATGAATCCCTCCCTTGTGATCCTCTCCGGAGTCCTGGCCAGTC ACTATATCCACATTGTCAAAGACGTCATTCGCCAGCAGGCCTTGTCCT CCGTGCAGGACGTGGATGTGGTGGTTTCGGATTTGGTTGACCCCGCC CTGCTGGGTGCTGCCAGCATGGTTCTGGACTACACAACACGCAGGAT CTACTAG | 2 |
| GNE mod1 | ATGGAAAAGAACGGCAACAACCGGAAGCTGAGAGTGTGCGTGGCCAC CTGTAACAGAGCCGACTACTCTAAGCTGGCCCCTATCATGTTCGGCAT CAAGACCGAGCCTGAGTTCTTCGAGCTGGACGTGGTGGTGCTGGGCA GCCACCTGATCGACGATTACGGCAACACCTACCGGATGATCGAGCAG GACGACTTCGACATCAACACCCGGCTGCACACCATCGTGCGGGGAGA AGATGAAGCCGCCATGGTGGAATCTGTCGGACTGGCCCTTGTGAAGC TGCCCGACGTGCTGAACAGACTGAAGCCCGACATCATGATCGTGCAC GGCGATAGATTCGACGCCCTGGCTCTGGCTACAAGCGCCGCTCTGAT | 3 |
| | GAACATCCGGATCCTGCACATCGAAGGCGGCGAAGTGTCTGGCACCA TCGACGACTCTATCCGGCACGCCATTACCAAACTGGCCCACTACCACG TGTGCTGCACAAGATCTGCCGAGCAGCACCTGATTAGCATGTGCGAG GACCACGACAGAATCCTGCTGGCCGGCTGTCCCAGCTACGATAAGCT GCTGAGCGCCAAGAACAAGGACTACATGAGCATCATCAGAATGTGGCT GGGCGACGACGTGAAGTCCAAGGATTACATCGTGGCCCTGCAGCACC CCGTGACCACCGATATCAAGCACAGCATCAAGATGTTCGAGCTGACCC TGGACGCCCTGATCAGCTTCAACAAGAGAACCCTGGTGCTGTTCCCCA ACATCGACGCCGGCAGCAAAGAAATGGTCCGAGTGATGCGGAAGAAG GGCATCGAGCATCACCCCAACTTCCGGGCCGTGAAGCACGTGCCCTT CGACCAGTTTATTCAGCTGGTGGCCCACGCCGGCTGCATGATCGGAA ATAGTAGCTGCGGCGTCAGAGAAGTGGGCGCCTTTGGAACCCCTGTG ATCAACCTGGGCACCAGACAGATCGGCAGAGAAACCGGCGAAAACGT GCTGCACGTGCGGGATGCCGACACACAGGATAAGATTCTGCAGGCCC TGCATCTGCAGTTCGGCAAGCAGTACCCCTGCTCCAAGATCTACGGC GACGGAAACGCCGTGCCTCGGATCCTGAAGTTTCTGAAGTCCATCGA CCTGCAAGAGCCCCTGCAGAAGAAGTTCTGCTTCCCACCTGTGAAAGA GAACATCAGCCAGGACATCGACCACATCCTGGAAACCCTGTCTGCCCT GGCTGTTGATCTCGGCGGCACCAATCTGAGAGTGGCCATCGTGTCTAT GAAGGGCGAGATCGTGAAGAAGTACACCCAGTTCAACCCCAAGACCT ACGAGGAACGCATCAACCTGATCCTGCAGATGTGCGTGGAAGCCGCC GCTGAAGCCGTGAAACTGAATTGCAGAATCCTCGGCGTGGGAATCAG CACCGGCGGCAGAGTGAATCCCAGAGAAGGCATCGTGCTGCATAGCA CCAAGCTGATCCAAGAGTGGAACAGCGTGGACCTGAGAACCCCTCTG AGCGACACACTGCATCTCCCTGTGTGGGTCGACAACGACGGCAATTG TGCCGCTCTGGCCGAGAGAAAGTTCGGCCAAGGCAAAGGCCTGGAAA ACTTCGTGACCCTGATCACCGGCACAGGCATCGGCGGAGGAATCATT CACCAGCACGAGCTGATTCACGGCAGCAGCTTTTGTGCTGCCGAGCT GGGCCATCTGGTGGTGTCTCTTGATGGCCCTGATTGCAGCTGTGGCT CCCACGGATGTATCGAGGCCTATGCCTCTGGCATGGCACTGCAGAGA GAGGCCAAGAAACTGCACGACGAGGACCTGCTGCTGGTGGAAGGCAT GAGCGTGCCAAAAGATGAGGCTGTGGGCGCCCTGCACCTTATTCAGG CTGCCAAACTGGGCAACGCCAAGGCTCAGAGCATCCTGAGAACAGCC GGAACAGCCCTCGGACTGGGCGTCGTGAATATTCTGCACACAATGAA CCCCAGCCTGGTCATCCTGTCTGGCGTGCTGGCCTCTCACTACATCCA CATCGTGAAGGACGTGATCCGGCAGCAGGCTCTGTCTAGCGTGCAGG ATGTCGACGTGGTCGTGTCCGATCTGGTGGATCCTGCTCTGCTGGGA GCCGCTTCTATGGTGCTGGACTACACCACACGGCGGATCTACTGA | |
| GNE mod2 | ATGGAGAAAACGGTAATAATAGGAAGCTTCGGGTCTGTGTTGCAACC TGCAATCGAGCAGATTACTCTAAGCTGGCACCCATAATGTTCGGGATA AAGACTGAGCCTGAATTCTTCGAACTCGATGTGGTAGTCCTGGGGAGT CACTTGATTGACGACTACGGTAATACTTATAGAATGATTGAACAAGATG ACTTTGACATAAACACCCGGCTGCATACGATCGTACGAGGGGAAGAC GAAGCTGCAATGGTAGAATCCGTAGGTTTGGCACTTGTAAAGTTGCCT GACGTCCTTAATCGCCTTAAACCGGACATTATGATTGTACATGGTGAC CGATTCGACGCGCTCGCACTTGCAACATCCGCGGCACTCATGAACATT AGGATACTTCACATTGAAGGAGGCGAAGTAAGTGGAACCATAGACGAT TCAATTAGACATGCCATTACCAAACTCGCCCACTACCACGTATGTTGTA CACGCTCCGCGGAGCAGCATCTTATAAGCATGTGCGAAGATCACGATA GGATCCTTCTTGCTGGCTGTCCAAGTTACGATAAGTTGCTTAGCGCCA AGAACAAAGACTACATGTCAATCATAAGAATGTGGTTGGGGGATGATG TAAAGAGCAAAGATTATATCGTCGCCTTGCAGCATCCAGTGACCACGG ACATCAAGCATTCAATCAAGATGTTCGAACTTACACTGGATGCCCTGAT TAGCTTTAACAAAAGAACCTTGGTATTGTTCCCGAACATAGATGCGGGT TCCAAAGAAATGGTGAGAGTTATGAGAAAAAAGGGTATCGAGCACCAT CCGAACTTTCGGGCCGTGAAACATGTTCCATTCGATCAATTCATTCAAC | 4 |
| | TGGTCGCGCATGCTGGTTGCATGATAGGCAATTCCTCATGTGGTGTCC GAGAGGTGGGGGCCTTCGGTACACCGGTTATTAACCTTGGTACAAGA CAGATTGGGCGCGAGACTGGCGAAAATGTTTTGCATGTGCGCGACGC GGATACGCAAGACAAAATACTTCAAGCATTGCATCTTCAGTTTGGCAA GCAATATCCCTGCAGCAAGATATACGGCGACGGAACGCAGTACCCA GGATTTTGAAATTCCTTAAGAGCATTGATCTTCAGGAACCACTGCAAAA GAAGTTTTGCTTCCCACCAGTAAAAGAAAATATCTCACAGGACATCGAT CACATACTGGAAACACTGAGCGCATTGGCTGTAGACCTGGGCGGTAC GAATTTGAGAGTGGCAATTGTTAGTATGAAAGGCGAGATAGTCAAGAA GTATACACAGTTTAATCCAAAAACATACGAAGAAAGAATAAATCTCATA CTTCAGATGTGCGTCGAAGCGGCGGCCGAGGCCGTCAAATTGAATTG CAGAATATTGGGGGTTGGTATAAGTACCGGGGGGAGAGTAAATCCCA GAGAGGGAATTGTTCTGCACTCAACGAAATTGATACAAGAATGGAACT CAGTGGACCTGAGAACCCCACTCAGTGACACACTCCATCTGCCGGTAT GGGTCGACAATGACGGCAACTGCGCGGCGCTTGCCGAAAGGAAGTTC GGTCAGGGTAAGGGGTTGGAGAATTTTGTGACTCTCATAACTGGCACT GGTATAGGAGGAGGAATCATCCATCAGCATGAGCTGATTCATGGATCA AGTTTCTGTGCTGCCGAACTGGGTCACCTCGTGGTGTCCTTGGATGGA CCGGACTGCAGCTGTGGCTCACATGGCTGCATCGAAGCTTACGCTAG CGGTATGGCTTTGCAGCGCGAGGCCAAAAAGCTGCACGACGAAGACC TCCTCTTGGTGGAGGGAATGTCAGTACCTAAGGACGAAGCGGTCGGG GCCCTTCACCTCATCCAGGCCGCTAAACTTGGAAACGCAAAAGCACAG TCCATCTTGCGGACTGCGGGAACGGCTCTCGGACTGGGTGTTGTCAA CATCTTGCACACAATGAACCCGAGCCTGGTCATCCTCAGTGGCGTGCT GGCGTCTCATTATATCCATATAGTCAAAGACGTAATACGACAACAGGCT CTCAGCAGCGTACAAGACGTCGATGTGGTTGTGTCCGATCTGGTAGAC CCTGCTCTCCTCGGGGCAGCCTCCATGGTTCTTGATTACACCACCAGG CGAATATATTAG | |
| GNE mod3 | ATGGAGAAGAACGGAAATAATAGAAAACTGCGGGTGTGCGTCGCCAC TTGCAACAGGGCCGATTACTCCAAGCTGGCCCTATCATGTTTGGGAT CAAGACTGAGCCCGAGTTCTTCGAGCTGGATGTGGTGGTGCTGGGCT CCCACCTGATCGACGACTACGGCAATACATACCGCATGATTGAGCAGG ACGACTTCGATATTAATACTAGACTGCACACCATTGTCAGGGGAGAGG ACGAGGCCGCCATGGTGGAAAGCGTGGGCCTGGCCCTGGTGAAGCT GCCTGATGTGCTGAATAGACTGAAGCCCGATATTATGATAGTGCACGG AGACAGGTTCGACGCCCTCGCCCTGGCCACCTCCGCCGCCCTGATGA ACATCAGAATCCTGCACATCGAAGGCGGCGAAGTGTCCGGCACTATT GATGACTCTATTAGGCACGCCATTACCAAGCTGGCCCACTATCACGTG TGTTGTACTAGGAGTGCCGAACAGCACCTGATTTCCATGTGCGAGGAC CATGACCGCATCCTGCTGGCAGGCTGTCCCAGCTACGACAAACTGCT GTCTGCTAAAAATAAAGATTATATGTCCATCATTAGAATGTGGCTGGGG GACGATGTGAAGAGCAAAGACTACATTGTGGCCCTCCAGCATCCAGTG ACCACTGACATTAAGCACTCCATTAAAATGTTCGAGCTGACTCTGGATG CCCTGATTTCCTTTAATAAAAGAACCCTGGTCCTGTTCCCAAACATTGA CGCCGGCAGTAAAGAAATGGTGAGGGTGATGCGCAAGAAAGGCATCG AGCACCACCCCAACTTTAGAGCCGTGAAGCACGTGCCCTTTGACCAGT TCATCCAGCTGGTCGCCCACGCCGGCTGCATGATTGGTAACTCCTCCT GCGGAGTGAGAGAGGTGGGCGCCTTTGGTACACCCGTGATCAATCTG GGCACACGGCAGATCGGCCGCGAGACCGGGGAAAACGTGCTGCATG TTAGGGACGCCGACACCCAGGATAAGATTCTGCAGGCTCTCCACCTG CAGTTCGGGAAGCAGTATCCTTGCAGCAAAATCTACGGCGACGGCAA CGCCGTGCCCAGAATCCTGAAGTTTCTGAAATCAATCGATCTGCAGGA GCCTCTGCAGAAGAAGTTCTGTTTCCCACCTGTGAAAGAGAACATCTC CCAGGACATTGATCACATCCTGGAGACACTGTCCGCCCTGGCTGTGG ACCTCGGGGGCACAAATCTGCGCGTGGCAATCGTGTCCATGAAAGGC GAGATCGTGAAAAAATATACCCAATTTAATCCTAAGACATACGAGGAGA | 5 |
| | GAATCAACCTGATCCTGCAGATGTGCGTGGAAGCCGCTGCTGAGGCC GTGAAGCTGAACTGTCGGATCCTGGGCGTGGGGATTAGCACCGGCGG CCGGGTGAACCCCCGAGAGGGCATCGTGCTGCACTCCACAAAGCTGA TCCAGGAGTGGAATAGCGTGGATCTGCGGACACCTCTGAGCGACACC CTGCATCTGCCCGTGTGGGTCGATAATGACGGAAACTGCGCCGCCCT GGCAGAAAGAAAATTTGGCCAGGGGAAGGGGCTCGAGAACTTTGTGA CTCTGATTACCGGCACCGGCATCGGCGGCGGAATCATCCACCAGCAC GAACTGATCCACGGGTCATCCTTCTGCGCCGCCGAACTGGGCCACCT GGTGGTGTCACTGGATGGCCCCGACTGTTCTTGCGGCAGCCACGGCT GTATCGAGGCCTATGCCAGTGGGATGGCCCTGCAGCGCGAAGCCAAG AAGCTGCACGACGAGGATCTGCTGCTGGTCGAGGGAATGAGCGTCCC AAAGGATGAGGCCGTGGGGGCCCTGCACCTGATTCAGGCTGCCAAGC TGGGGAACGCCAAGGCCCAGTCTATTCTTCGCACAGCTGGCACAGCA CTGGGCCTGGGAGTGGTGAACATCCTCCACACAATGAACCCCTCTCT GGTGATCCTGAGCGGCGTGCTGGCTAGCCATTACATCCACATCGTGA AGGATGTGATCAGGCAGCAGGCCCTGAGCAGCGTGCAGGATGTGGAC GTAGTGGTGTCCGACCTGGTGGACCCTGCCCTGCTGGGCGCCGCCTC CATGGTGCTGGACTACACCACAAGGCGGATCTACTAA | |
| GNE mod4 | ATGGAAAAGAACGGTAATAACAGAAAGCTCAGAGTGTGTGGCCACA TGCAACCGGGCCGACTACAGCAAGCTGGCCCCTATCATGTTCGGCAT TAAAACCGAGCCTGAGTTCTTCGAGCTGGACGTGGTGGTGCTGGGCA GCCACCTGATCGATGACTATGGCAACACCTACAGAATGATCGAGCAGG ACGATTTCGACATCAACACCAGACTGCATACAATCGTCCGAGGAGAAG ATGAGGCCGCCATGGTGGAAAGCGTGGGCCTGGCCCTGGTCAAGCT GCCTGACGTTCTCAACCGGCTGAAGCCCGATATCATGATCGTGCACG GCGATCGGTTCGACGCCCTGGCTCTGGCTACAAGCGCCGCCCTGATG AACATCAGAATCCTGCATATCGAAGGCGGCGAGGTGTCCGGCACCAT CGACGACAGCATCCGCCACGCCATCACCAAGTTAGCTCACTACCACGT GTGCTGCACCAGGTCCGCCGAGCAGCACCTGATCAGCATGTGCGAGG ACCACGATAGAATCCTGCTGGCTGGCTGCCCCAGCTACGACAAGCTG CTCAGCGCCAAAAACAAAGACTACATGTCTATCATCCGGATGTGGCTG GGCGACGACGTGAAGTCCAAAGACTATATAGTGGCTCTGCAACACCCT GTGACAACCGACATTAAGCACAGCATCAAAATGTTCGAACTGACCCTG GACGCCCTCATCTCTTTCAACAAGCGGACACTGGTGCTGTTCCCAAAC ATCGACGCCGGATCTAAAGAGATGGTCAGAGTGATGAGAAAGAAGGG GATCGAGCACCACCCCAACTTTAGAGCCGTGAAACACGTGCCATTCGA TCAGTTTATCCAGCTGGTGGCCCACGCCGGCTGTATGATCGGAAACA GCAGCTGTGGCGTGCGGGAAGTGGGCGCCTTTGGCACCCCTGTGATC AACCTGGGAACCCGGCAGATCGGCCGGGAGACAGGAGAGAACGTGC TGCACGTTAGAGATGCCGATACCCAGGATAAGATCCTGCAGGCCCTG CACCTGCAGTTCGGCAAACAGTACCCTTGTAGCAAGATCTACGGCGAC GGCAATGCCGTGCCCAGAATCCTGAAGTTCCTGAAAAGCATCGACCTG CAGGAGCCACTGCAAAAGAAGTTTTGCTTCCCCCCCGTGAAGGAAAAT ATCAGCCAGGACATCGACCACATCCTGGAAACACTGTCTGCCCTGGC CGTGGACCTGGGCGGCACAAACCTGCGGGTGGCCATCGTGTCCATGA AGGGCGAAATCGTCAAAAAGTACACACAGTTCAACCCCAAGACATACG AGGAAAGAATCAATCTGATACTTCAGATGTGTGTTGAAGCCGCTGCTG AAGCCGTGAAGCTGAACTGCAGAATCCTGGGCGTGGGCATCTCTACA GGCGGCAGAGTGAACCCTAGGGAGGGCATCGTGCTGCATTCTACCAA GCTGATTCAAGAGTGGAACAGTGTTGATCTGAGAACCCCTCTGAGCGA CACCCTGCACTTGCCTGTGTGGGTGGACAACGACGGCAATTGCGCCG CCCTGGCCGAGCGGAAGTTTGGACAGGGCAAGGGCCTGGAAAACTTC GTGACCCTGATCACCGGCACCGGAATCGGCGGCGGCATTATCCACCA GCACGAGCTGATCCACGGCAGCAGCTTCTGCGCCGCTGAACTGGGCC ACCTGGTCGTGTCTCTGGATGGTCCTGATTGCAGCTGCGGCAGCCAC GGCTGCATCGAGGCCTACGCCAGCGGAATGGCCCTGCAAAGAGAAGC | 6 |
| | TAAGAAGCTGCACGACGAGGACCTGCTGCTGGTTGAGGGCATGAGCG TGCCTAAGGACGAGGCCGTGGGCGCCCTACACCTGATCCAGGCCGCT AAGCTGGGCAACGCCAAGGCCCAGAGCATCCTGAGAACAGCCGGCAC CGCCCTGGGCCTGGGCGTGGTCAACATCCTCCATACCATGAATCCTA GCCTGGTGATTCTGAGCGGCGTGCTGGCCAGCCACTACATCCACATC GTGAAGGATGTGATCCGGCAGCAGGCCCTGTCCTCTGTGCAGGATGT GGACGTGGTGGTGTCGGACCTGGTGGATCCTGCTCTCCTGGGAGCCG CATCTATGGTGCTGGACTACACCACCCGCAGAATCTACTGA | |

### 1-2. Comparison of GNE Nucleotide Sequences

In the present Experimental Example, the nucleotide sequence of the GNE gene included in the expression cassette produced in Experimental Example 1-1 were specifically compared, and the results are as shown in Table 2 below.

**[Table 2]**

| **Name** | **Nucleotide Sequence Composition** | | | | | **Sequence Similarity to GNE wt** | |
|---|---|---|---|---|---|---|---|
| | **A** | **C** | **G** | **T** | **G-C** | **Similarity (bp)** | **Similarity (%)** |
| GNE wt | 26.79 % (581 bp) | 22.08 % (479 bp) | 25.27 % (548 bp) | 25.86 % (561 bp) | 47.35 % (1,027 bp) | - | - |
| GNE mod1 | 23.88 % (518 bp) | 29.18 % (633 bp) | 28.72 % (623 bp) | 18.21 % (395 bp) | 57.91 % (1,256 bp) | 1,673 / 2,169 | 77 % |
| GNE mod2 | 28.26 % (613 bp) | 22.36 % (485 bp) | 25.96 % (563 bp) | 23.42 % (508 bp) | 48.32 % (1,048 bp) | 1,619 / 2,169 | 75 % |
| GNE mod3 | 23.70 % (514 bp) | 27.43 % (595 bp) | 29.00 % (629 bp) | 19.87 % (431 bp) | 56.43 % (1,224 bp) | 1,668 / 2,169 | 77 % |
| GNE mod4 | 24.30 % (527 bp) | 28.82 % (625 bp) | 28.72 % (623 bp) | 18.17 % (394 bp) | 57.54 % (1,248 bp) | 1,678 / 2,169 | 77% |

As shown in Table 2 above, the codon optimized nucleotide sequences (GNE mod1, GNE mod2, GNE mod3, GNE mod4) according to an embodiment were confirmed to exhibit sequence similarity/identity of about 75 to 77 % compared to the wild-type GNE, and GC-content (%) in the range of 48.32 to 57.91 %.

### Experimental Example 2. Evaluation of GNE Protein Expression Level Using Recombinant Plasmid Vector

In the present Experimental Example, the recombinant plasmid vector into which the expression cassette of Experimental Example 1 was inserted was transfected into HEK293T cells and C2C12 cells, respectively, and the expression level of the GNE protein was evaluated. Specifically, HEK293T cells were seeded at 5.0E+05 cells/6-well, and C2C12 cells were seeded at 1.25E+05 cells/6-well, on the following day, 2.5 µg each of four recombinant plasmid vectors including codon optimized GNE nucleotide sequences (Example 1 (GNE mod1), Example 2 (GNE mod2), Example 3 (GNE mod3), and Example 4 (GNE mod4)) were transfected into the respective cells. After culturing the above transfected cells for 48 hours, proteins were extracted from the cells and GNE protein expression was confirmed by western blotting. In addition, as a negative control group, a group that was not subjected to transfection was used, and as a comparison group, a group transfected with a recombinant plasmid vector administered with a wild-type GNE nucleotide sequence was used.

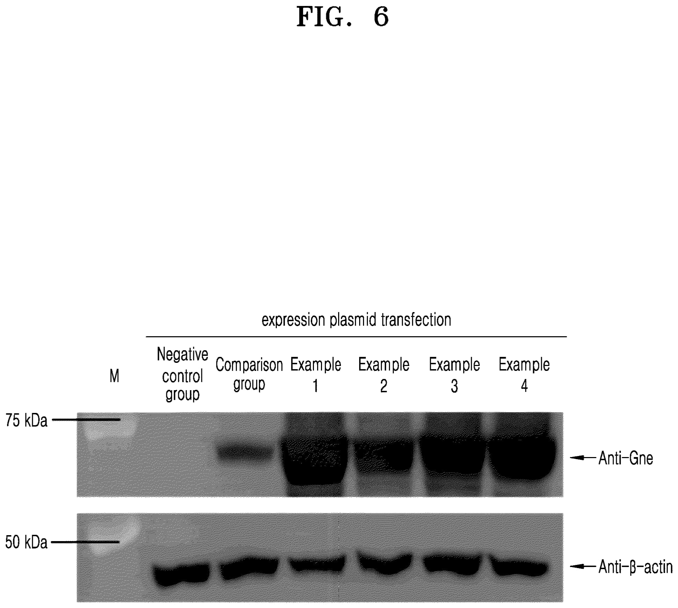

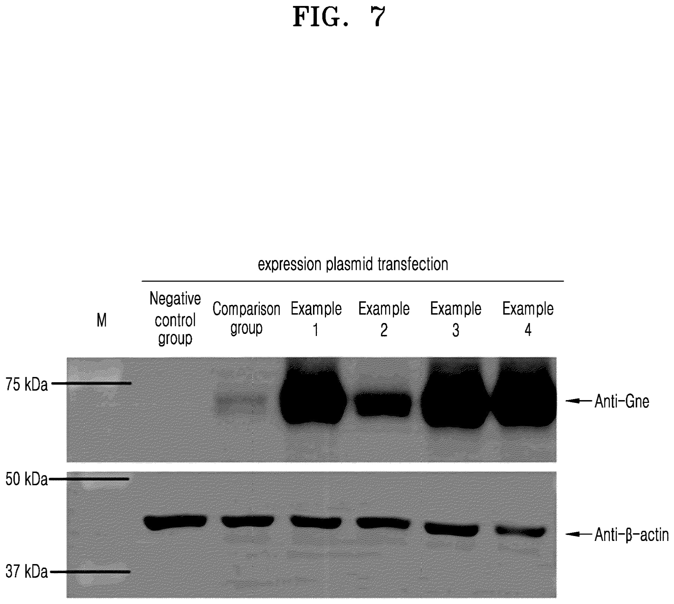

As a result, as shown in Table 3, Table 4, FIG. 6 and FIG. 7, it was confirmed that the group introduced with the recombinant plasmid vector including the GNE nucleotide sequence according to an embodiment (Example 1 to Example 4) exhibited a significantly increased the expression level of the GNE protein compared to the control group and the comparison group.

**[Table 3]**

| **HEK293T** | **GNE** | | **β-actin** | | **GNE/β-actin** |
|---|---|---|---|---|---|
| Negative Control Group | 0 | - | 25715.64 | - | - |
| Comparison Group | 6408.497 | 1.00 | 27665.3 | 1.00 | 1.00 |
| Example 1 | 30494.23 | 4.758405 | 21721.88 | 0.785167 | 6.06 |
| Example 2 | 21371.98 | 3.334945 | 24103.95 | 0.87127 | 3.83 |
| Example 3 | 29208.05 | 4.557707 | 27060.59 | 0.978142 | 4.66 |
| Example 4 | 30236.93 | 4.718257 | 24548.95 | 0.887356 | 5.32 |

**[Table 4]**

| **C2C12** | **GNE** | | **β-actin** | | **GNE/β-actin** |
|---|---|---|---|---|---|
| Negative Control Group | 0 | - | 24780.64 | - | - |
| Comparison Group | 3672.355 | 1.00 | 20894.98 | 1.00 | 1.00 |
| Example 1 | 24323.59 | 6.623431 | 20647.35 | 0.988149 | 6.70 |
| Example 2 | 11514.79 | 3.135533 | 21266.23 | 1.017767 | 3.08 |
| Example 3 | 25866.28 | 7.043512 | 20908.23 | 1.000634 | 7.04 |
| Example 4 | 24471.96 | 6.663833 | 16353.81 | 0.782667 | 8.51 |

### Experimental Example 3. Evaluation of GNE Protein Expression Levels Using Recombinant AAV9 Vectors

In the present Experimental Example, the recombinant AAV9 vector into which the expression cassette of Experimental Example 1 was inserted was transduced into HEK293T cells and C2C12 cells, respectively, and the expression level of the GNE protein was evaluated. Specifically, HEK293T cells were seeded at 5.0E+05 cells/6-well, and on the following day, four types of recombinant AAV9 vectors including codon optimized GNE nucleotide sequences (Example 1 (GNE mod1), Example 2 (GNE mod2), Example 3 (GNE mod3), and Example 4 (GNE mod4)) were transduced into the cells at a level of 5.0E+05 VG (vector genome)/cell, respectively. After 24 hours post-transduction, the medium was replaced, and the transduced cells were cultured for an additional 24 hours. Additionally, after C2C12 cells were seeded at 6.25E+04 cells/6-well, the cells were transduced with 1.0E+03 pfu/cell of adenovirus and four types of recombinant AAV9 vectors (Example 1 (GNE mod1), Example 2 (GNE mod2), Example 3 (GNE mod3), Example 4 (GNE mod4)) at 1.0E+06 VG (vector genome)/cell, respectively. After 6 hours post-transduction, the medium was replaced, and the transduced cells were cultured for an additional 66 hours. Afterwards, proteins were extracted from the HEK293T and C2C12 cells and GNE protein expression was confirmed by western blotting. In addition, as a negative control group, a group that was not subjected to transfection was used, and as a comparison group, a group transduced with a recombinant AAV9 vector administered with a wild-type GNE nucleotide sequence was used.

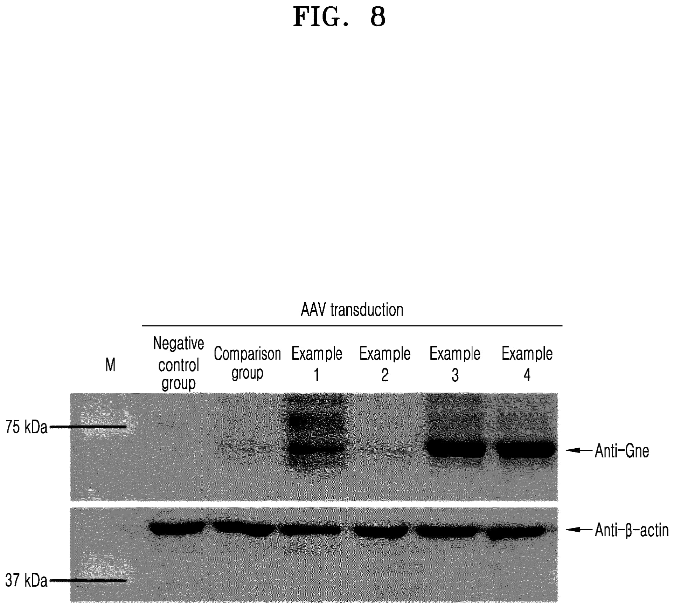

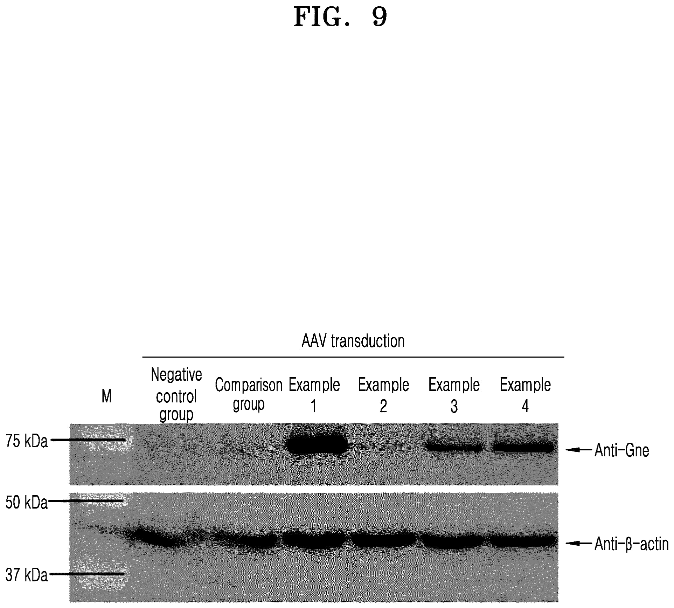

As a result, as shown in Table 5, Table 6, FIG. 8 and FIG. 9, it was confirmed that the GNE protein expression level of the group introduced with the recombinant AAV9 vector including the GNE nucleotide sequence according to an embodiment (Example 1 to Example 4) was similar to or enhanced compared to the comparison group. In particular, among the groups introduced with the recombinant AAV9 vector according to an embodiment, the effect of enhancing GNE protein expression in Examples 1, 3, and 4, in which the GC-content (%) was relatively higher than that of the wild-type, was remarkable.

**[Table 5]**

| **HEK293T** | **GNE** | | **β-actin** | | **GNE/β-actin** |
|---|---|---|---|---|---|
| Negative Control Group | 0 | - | 23790.05 | - | - |
| Comparison Group | 4423.205 | 1.00 | 25836 | 1.00 | 1.00 |
| Example 1 | 31730.87 | 7.173729 | 20818.95 | 0.805812 | 8.90 |
| Example 2 | 4865.083 | 1.0999 | 21852.59 | 0.845819 | 1.30 |
| Example 3 | 36549.22 | 8.263062 | 24805.35 | 0.960108 | 8.61 |
| Example 4 | 32655 | 7.382657 | 23293.13 | 0.901576 | 8.19 |

**[Table 6]**

| **C2C12** | **GNE** | | **β-actin** | | **GNE/β-actin** |
|---|---|---|---|---|---|
| Negative Control Group | 10068.74 | - | 31175.37 | - | - |
| Comparison Group | 9394.891 | 1.00 | 26700.62 | 1.00 | 1.00 |
| Example 1 | 26386.3 | 2.808579 | 27251.74 | 1.020641 | 2.75 |
| Example 2 | 8902.184 | 0.947556 | 27748.23 | 1.039235 | 0.91 |
| Example 3 | 15369.69 | 1.635963 | 25656.91 | 0.960911 | 1.70 |
| Example 4 | 18049.33 | 1.921185 | 24812.15 | 0.929273 | 2.07 |

### Experimental Example 4. Evaluation of the Restoration Effect of GNE Expression Using GNE Knockdown Cell Lines

In the present Experimental Example, to evaluate the restoration effect of GNE expression, a C2C12 (mouse, myoblast) cell line with knocked-down mGne expression was used. An mGne knock down C2C12 cell line was established using a lentivirus including mGne shRNA inserted into C2C12 cells. Thereafter, two recombinant AAV9 vectors including codon optimized GNE nucleotide sequences (Example 3 (GNE mod3) and Example 4 (GNE mod4)) were transduced into cells at a level of 1E+6 VG (vector genome)/cell, respectively. In addition, a C2C12 cell line in which mGne was not knocked down (C2C12 NC) was used as a control group, a group in which no transduction was performed on the mGne knock down C2C12 cell line (C2C12 shGne #1 NC) was used as a negative control group, and a group in which a recombinant AAV9 vector with the wild-type GNE nucleotide sequence applied to the mGne knock down C2C12 cell line was introduced (C2C12 shGne #1 AAV-GNE (wt)) was used as a comparison group.

### 4-1. Evaluation of GNE Protein Expression Level

In the same manner as in Experimental Example 3 above, proteins were extracted from the mGne knock down C2C12 cell line and GNE protein expression was confirmed by western blot.

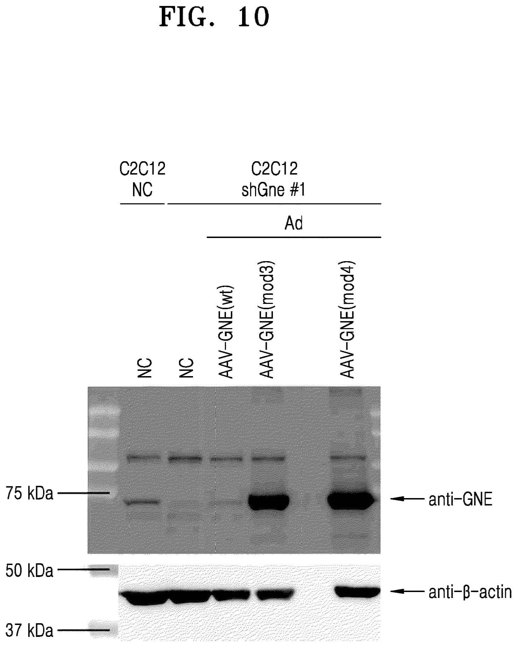

As a result, as shown in Table 7 and FIG. 10, the mGne knock down C2C12 cell line in the present Experimental Example showed a significantly reduced GNE expression level compared to the control group, indicating that the Gne knock down C2C12 cell line was stably established. In addition, despite introduction of a recombinant AAV9 vector including the GNE nucleotide sequence in the comparison group, GNE expression levels were not restored, in the group introduced with a recombinant AAV9 vector including a GNE nucleotide sequence (Example 3 and Example 4) according to an embodiment, the GNE expression level was not only recovered, but also confirmed to be enhanced above the expression level of the control group.

**[Table 7]**

| **C2C12 K/d** | **GNE** | | **β-actin** | | **GNE/β-actin** |
|---|---|---|---|---|---|
| Control Group | 3895.841 | - | 32147.33 | - | - |
| Negative Control Group | 1069.092 | - | 29333.35 | - | - |
| Comparison Group | 1787.698 | 1.00 | 21196.47 | 1.00 | 1.00 |
| Example 3 | 27241.52 | 15.23832 | 18854.23 | 0.889498 | 17.13 |
| Example 4 | 34146.47 | 19.1008 | 25930.42 | 1.223337 | 15.61 |

### 4-2. Evaluation of Sialic Acid Level

For each sample, SNA staining was performed for sialic acid (N-Acetylneuraminic acid: Neu5AC) and PNA staining was performed for galactose. Subsequently, flow cytometry (FACS) was performed to quantitatively evaluate the levels of Neu5AC-expressing cells (SNA-FITC) and Galactose-expressing cells (PNA-FITC).

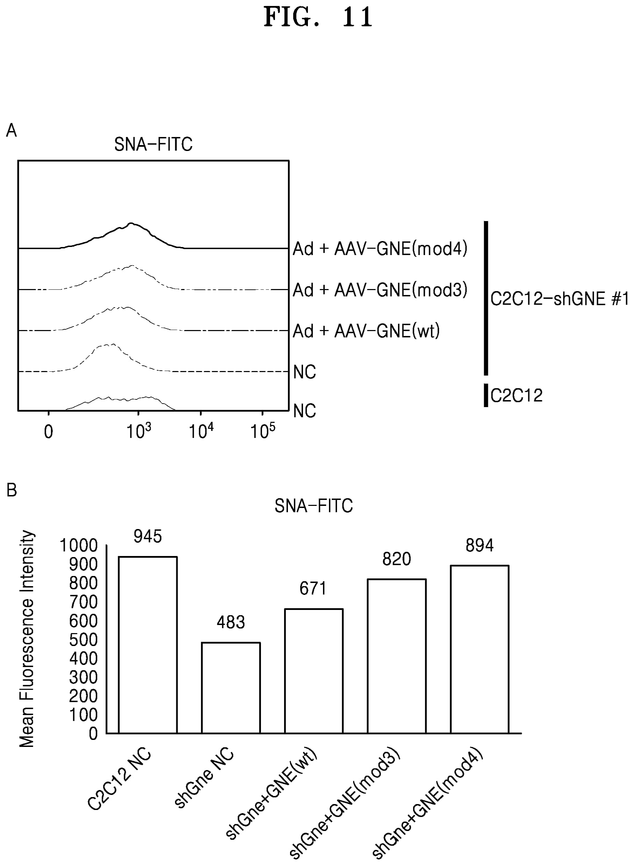

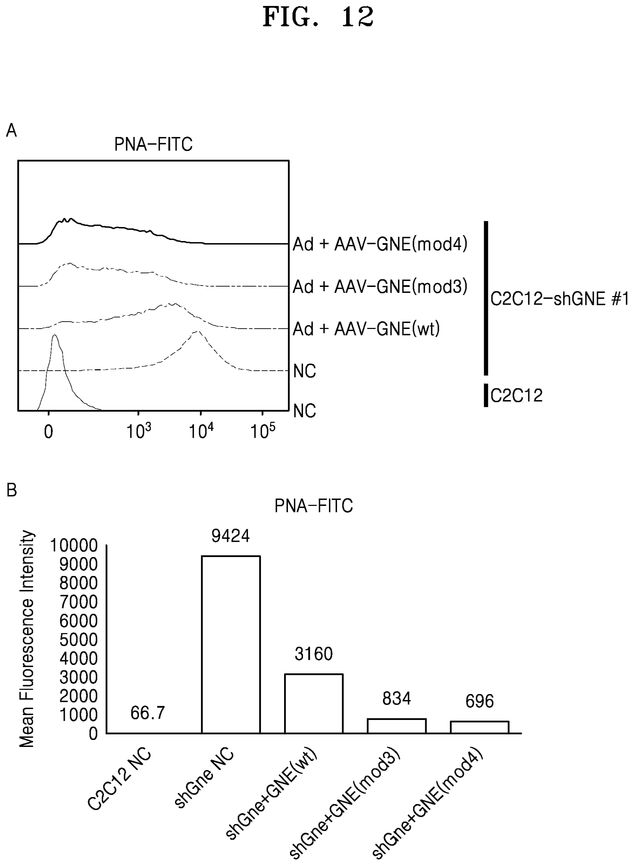

As a result, as shown in FIG. 11, in the mGne knock down C2C12 cell line in the present Experimental Example, the level of Neu5AC-expressing cells decreased, whereas in the group introduced with the recombinant AAV9 vector including the GNE nucleotide sequence (Example 3 and Example 4) according to an embodiment, the level of Neu5AC-expressing cells was confirmed to have recovered to a level similar to that of the control group. In addition, as shown in FIG. 12, in the mGne knock down C2C12 cell line in the present experimental example, the level of Galactose-expressing cells was significantly increased, whereas in the group introduced with the AAV9 vector according to an embodiment, it was confirmed that, as above, it was recovered to a level similar to that of the control group.

### Experimental Example 5. Evaluation of GNE Expression Recovery Effect by Administration of Recombinant AAV9 Vector in Disease Mouse Model

In this experimental example, the aim was to confirm the restoration of GNE expression by administration of a recombinant AAV9 vector through evaluation using a disease mouse model (GNE^{C44S}). To this end, 12- or 13-week-old C44S knock-in mice were selected as a disease mouse model, and recombinant AAV9 vectors with wild-type GNE nucleotide sequences applied at different doses, in other words, Gne KI LD (low dose) hGNE AAV, Gne KI MD (mid dose) hGNE AAV, and Gne KI HD (high dose) hGNE AAV, were administered to the disease mouse model. Thereafter, after 4 weeks post-administration, muscle tissue was obtained from the disease mouse model, and the expression level of GNE or related factors in the tissue was evaluated. In addition, in the present experimental example, normal mice (WT) were used as a control group, and a group administered Mock AAV to a disease mouse model (Gne KI Mock AAV) was used as a negative control group. The mouse model and administration conditions in the present experimental example are as shown in Table 8 below.

**[Table 8]**

| **Group** | | **Age** | **Number** | **Dose (per 200** *µℓ***)** |
|---|---|---|---|---|
| **Normal** | **WT** | 13-week-old (male) | 5 | PBS |
| **Gne C44S KI** | **Mock** | 12-week-old (male) | 3 | 1E+12 |
| | **LD hGNE AAV** | 13-week-old (male) | 4 | 1E+11 |
| | **MD hGNE AAV** | 13-week-old (male) | 4 | 3.3E+11 |
| | **HD hGNE AAV** | 12/13-week-old (male) | 4 | 1E+12 |

### 5-1. Evaluation of Sialic Acid Level

After obtaining muscle tissue from a disease mouse model administered with a recombinant AAV9 vector including a wild-type GNE nucleotide sequence, the muscle tissue was rapidly frozen in isopentane, sectioned, and slides were prepared, and air-dried at room temperature for 20 minutes. Acetone fixation was performed at -20 °C for 10 minutes, then washed with PBS, and for each sample, SNA staining was performed for N-Acetylneuraminic acid (Neu5AC) and PNA staining was performed for galactose. Afterwards, the cells were washed three times with PBS for 3 minutes each time, and the expression levels of Neu5AC and Galactose were evaluated.

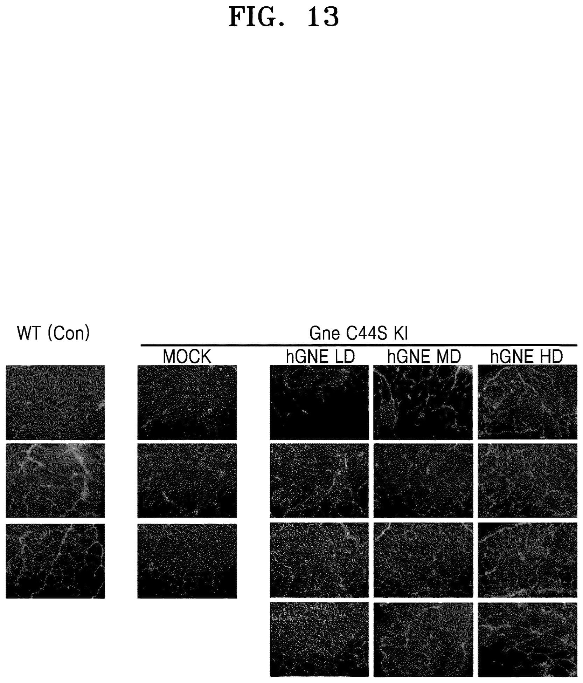

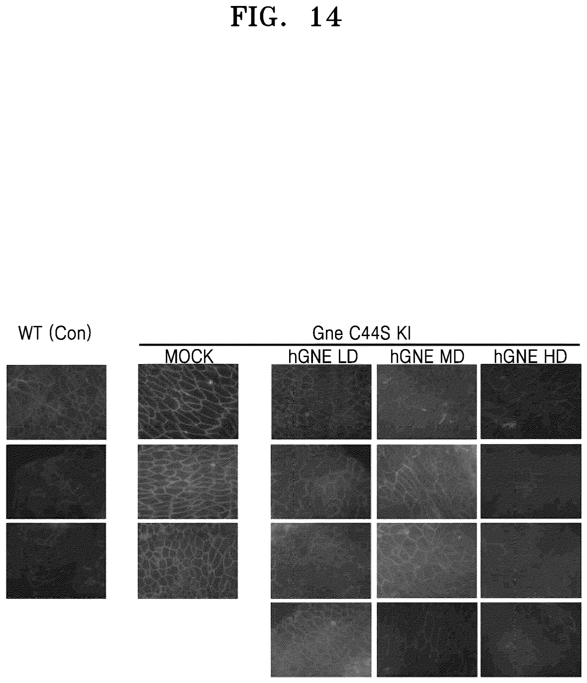

As a result, as shown in FIG. 13, in the mouse models administered with the recombinant AAV9 vector (Gne KI LD hGNE AAV, Gne KI MD hGNE AAV, Gne KI HD hGNE AAV), it was confirmed that the reduced expression level of Neu5AC caused by the lack of the GNE gene was restored in the tibialis anterior muscle of the mouse model. In addition, as shown in FIG. 14, in the mouse models administered with the recombinant AAV9 vector (Gne KI LD hGNE AAV, Gne KI MD hGNE AAV, Gne KI HD hGNE AAV), it was confirmed that the increased expression level of galactose resulting from the lack of the GNE gene was restored to the level of the control group in the tibialis anterior muscle of the mouse model. Notably, this restoration level showed a tendency to be dependent on the administered dose.

Additionally, at 4 weeks after administration of the recombinant AAV9 vectors including the wild-type GNE nucleotide sequence at different doses to the disease mouse model, the sialic acid levels were re-evaluated by performing the aforementioned SNA staining and PNA staining on the gastrocnemius muscle of the mouse model to re-evaluate the sialic acid levels.

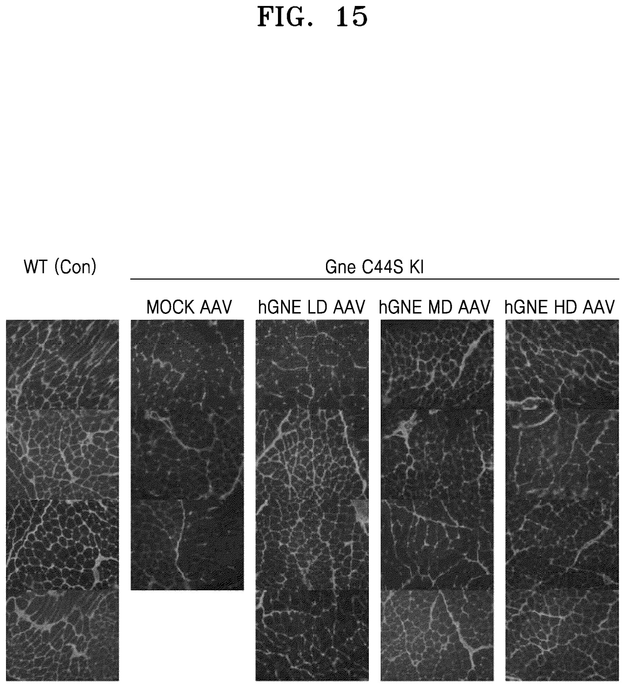

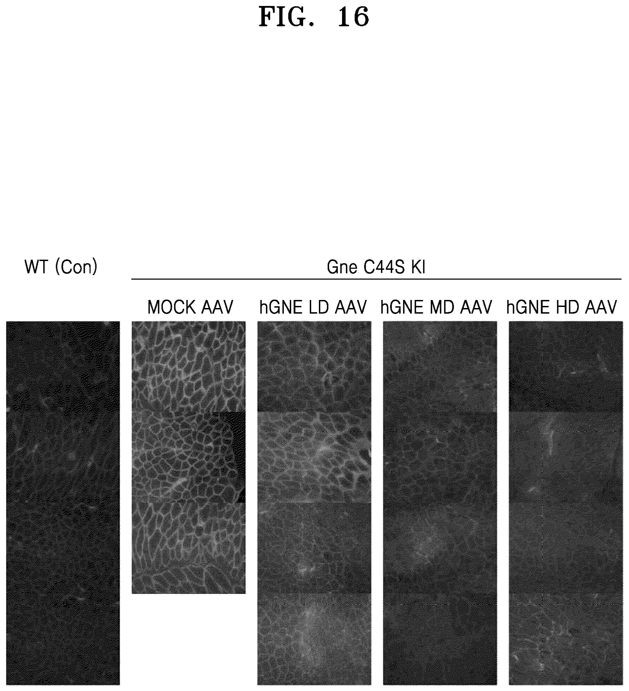

As a result, as shown in FIG. 15 and FIG. 16, it was confirmed that the reduced expression level of Neu5AC resulting from the lack of the GNE gene was restored by the administration of the recombinant AAV9 vector according to an embodiment, and the increased expression level of Galactose was also restored to the level of the control group by the administration of the recombinant AAV9 vector according to an embodiment.

That is, as shown in FIG. 13 to FIG. 16, recovery of expression levels was confirmed in different muscles, confirming recovery of sialic acid levels throughout the muscles of the mouse model.

### 5-2. Evaluation of GNE mRNA Level

After obtaining muscle tissue from a disease mouse model administered with a recombinant AAV9 vector including a wild-type GNE nucleotide sequence, the level of GNE mRNA in each sample was measured by q-RT PCR and quantitatively compared by normalization using a T-test.

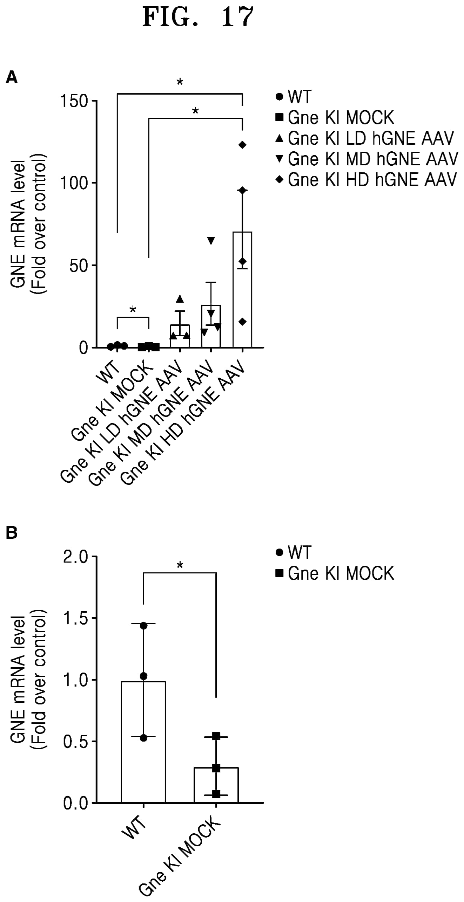

As a result, as shown in FIG. 17, in the negative control group (Gne Kl Mock) administered with Mock AAV to the disease mouse model, the level of GNE mRNA was reduced compared to the control group, whereas in the disease mouse model administered with the recombinant AAV9 vector including the GNE nucleotide sequence (Gne KI LD hGNE AAV, Gne KI MD hGNE AAV, Gne KI HD hGNE AAV), the level of GNE mRNA was increased, and this effect showed a tendency to be dependent on the administered dose.

### 5-3. Evaluation of GNE protein Expression Level

After obtaining muscle tissue from a disease mouse model administered with an recombinant AAV9 vector including a wild-type GNE nucleotide sequence, the level of GNE protein expression in each sample was confirmed twice by Western blotting (detection antibody: #NBP1-81621, #25079-1-ap).

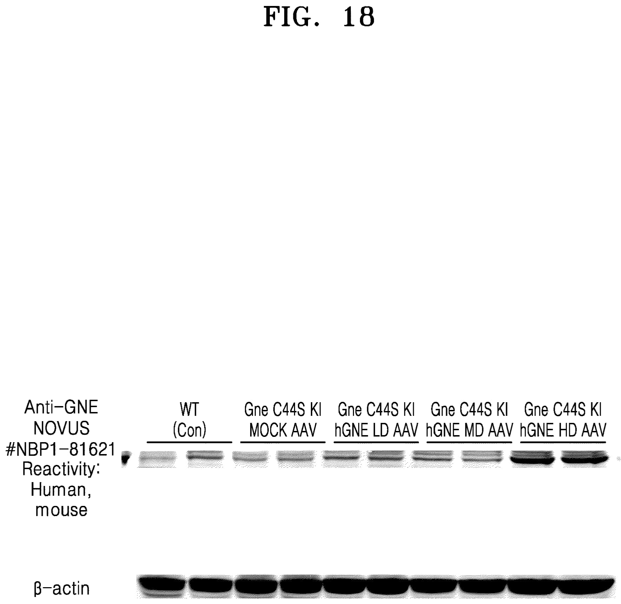

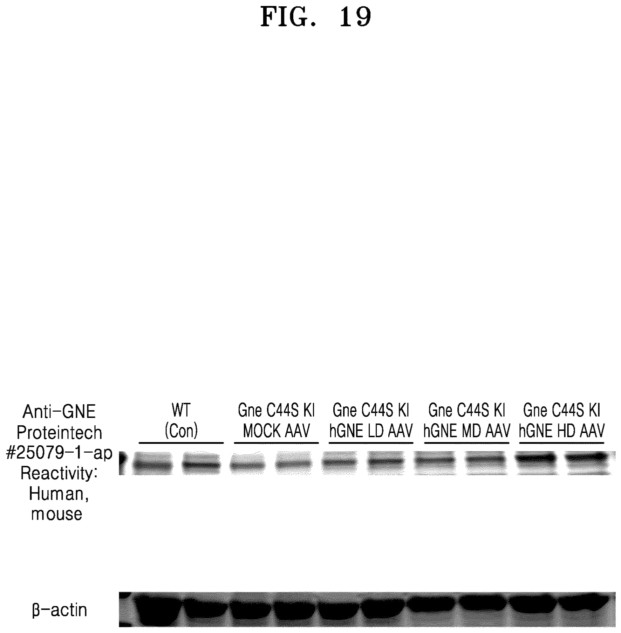

As a result, as shown in FIG. 18 and FIG. 19, the expression level of GNE protein was increased in the disease mouse models (Gne KI LD hGNE AAV, Gne KI MD hGNE AAV, Gne KI HD hGNE AAV) administered with the recombinant AAV9 vector, and in particular, this effect showed a tendency to be dependent on the administered dose.

### Experimental Example 6. Functional Recovery Evaluation Using Disease Mouse Model

In this experimental example, the aim was to confirm functional recovery by administration of a recombinant AAV9 vector through behavioral evaluation using a disease mouse model (GNE^{C44S}). To this end, C44S knock-in mice over 40 weeks old were selected as a disease mouse model, and a recombinant AAV9 vector including a GNE nucleotide sequence according to an embodiment (Example 3 (Gne KI GNE mod3 AAV), Example 4 (Gne KI GNE mod4 AAV)) was administered to the disease mouse model. Thereafter, after 4 weeks post-administration, treadmill tests and grip strength tests were conducted on subjects of each group. Along with the aforementioned behavioral evaluation, at 8 weeks post-administration, the level of sialic acid and level of GNE protein expression were confirmed in muscle tissues of each group. In addition, in the present experimental example, normal mice (C57BL/6) were used as a control group, a group administered Mock AAV9 to a disease mouse model (Gne KI Mock AAV) as a negative control group, and a group administered recombinant AAV9 with a wild-type GNE nucleotide sequence applied to a disease mouse model (Gne KI hGNE AAV) as a comparison group. The mouse model and administration conditions in the present experimental example are as shown in Table 9 below.

**[Table 9]**

| **Group** | | **Age** | **Number** | **Dose (per 200** *µℓ*) |
|---|---|---|---|---|
| **Normal** | **C57BL/6** | 40-week-old or older (male) | 5 | PBS |
| **Gne C44S KI** | **Mock** | 40-week-old or older (male) | 3 | 3.3E+11 |
| | **hGNE AAV** | 40-week-old or older (male) | 3 | 3.3E+11 |
| | **GNE mod3 AAV** | 40-week-old or older (male)) | 4 | 3.3E+11 |
| | **GNE mod4 AAV** | 40-week-old or older (male) | 4 | 3.3E+11 |

### 6-1. Evaluation of Motor Function Through Treadmill Testing

A treadmill test was conducted on a disease mouse model administered with a recombinant AAV9 vector including GNE nucleotide sequences according to an embodiment. Specifically, a pre-test was conducted for the first 5 minutes at a slope of 10 degrees and a speed of 5 m/min, after which the starting speed was set to 10 m/min and the speed was increased by 1 m/min every minute, up to a maximum speed of 25 m/min. When the mice reached the limit of their motor performance and stopped running, they were prompted to resume running by applying mild electric stimulation. Afterwards, if the subject failed to run after receiving an electric shock for more than 5 seconds, the test was terminated and the travel distance (m) was measured.

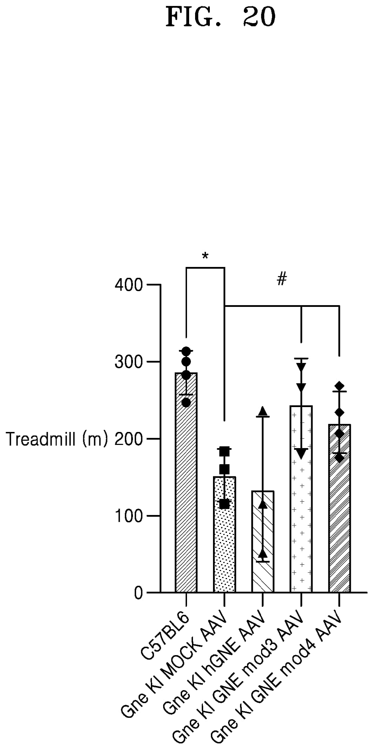

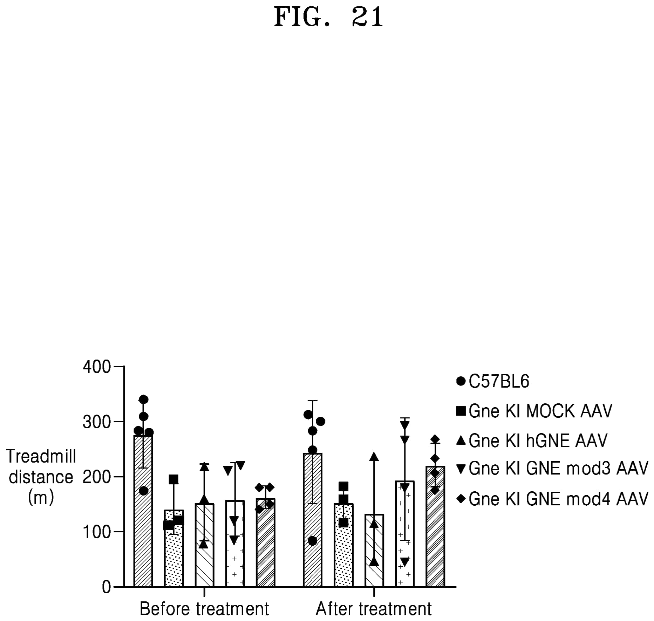

As a result, as shown in FIG. 20 and FIG. 21, it was confirmed that in the negative control group in which Mock AAV was administered to the disease mouse model and the comparison group in which the wild-type GNE nucleotide sequence was applied, the movement distance of the treadmill was reduced due to the lack of the GNE gene, whereas in the group in which the recombinant AAV9 vector including the GNE nucleotide sequence according to an embodiment was introduced, the travel distance of the treadmill was increased.

### 6-2. Evaluation of Motor Function Through Grip Strength Testing

A grip strength test was conducted on a disease mouse model administered with a recombinant AAV9 vector including GNE nucleotide sequences according to an embodiment. Specifically, the grip strength was measured by placing all four paws of the mouse on a grid and then pulling the tail to measure the gripping strength. Afterwards, the value of the gripping strength/body weight (g) was calculated and the average record was measured after 5 repetitions.

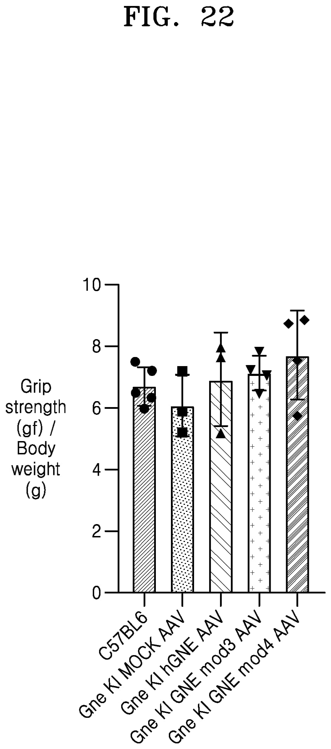

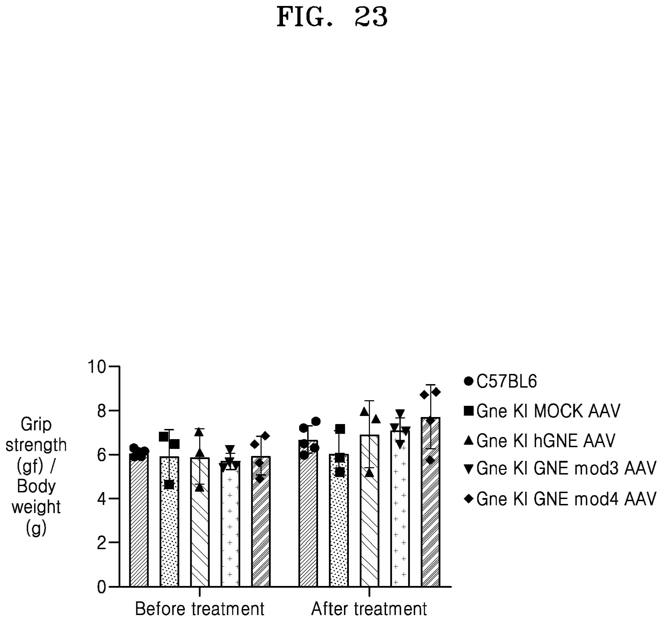

As a result, as shown in FIG. 22 and FIG. 23, similar to the above results, it was confirmed that the grip strength was enhanced in the group introduced with the recombinant AAV9 vector including the GNE nucleotide sequence according to an embodiment.

### 6-3. Evaluation of Sialic Acid Level

In a disease mouse model administered with a recombinant AAV9 vector including a GNE nucleotide sequence according to an embodiment, at 8 weeks post-administration, the expression levels of Neu5AC and Galactose were confirmed, respectively, through SNA staining and PNA staining of muscle tissues of each group according to the method of Example 5-1.

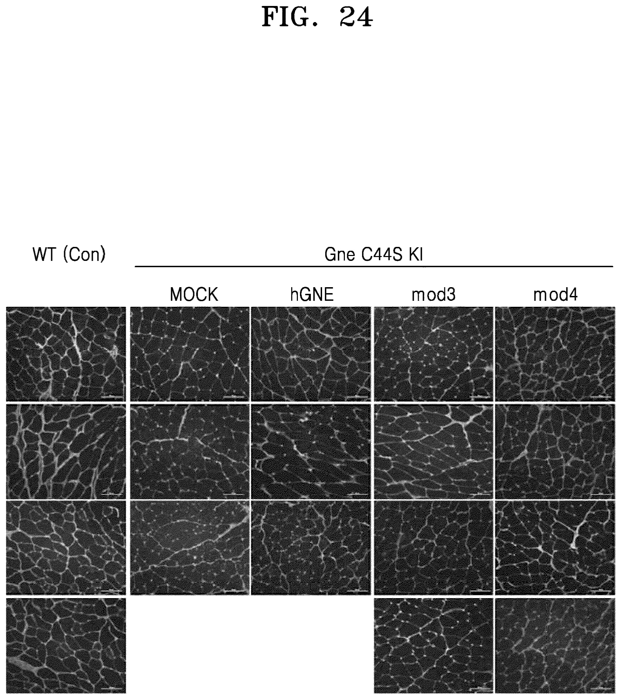

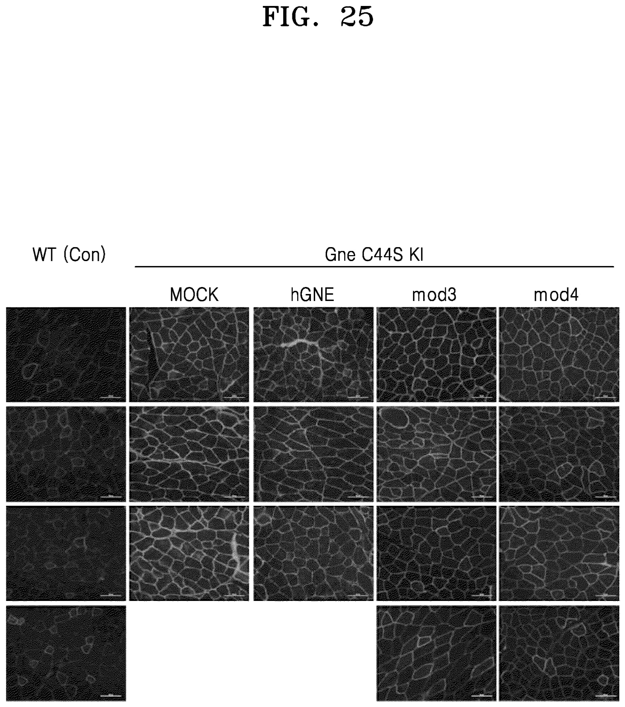

As a result, as shown in FIG. 24 and FIG. 25, it was confirmed that the reduced expression level of Neu5AC was restored in the group introduced with the recombinant AAV9 vector including the GNE nucleotide sequence according to an embodiment, and the increased expression level of Galactose was also restored to the level of the control group.

### 6-4. Evaluation of GNE protein Expression Level

In a disease mouse model administered with a recombinant AAV9 vector including a GNE nucleotide sequence according to an embodiment, at 8 weeks post-administration, the expression level of the GNE protein was confirmed by western blotting (detection antibody: #25079-1-ap) on muscle tissues of each group according to the method of Example 5-3.

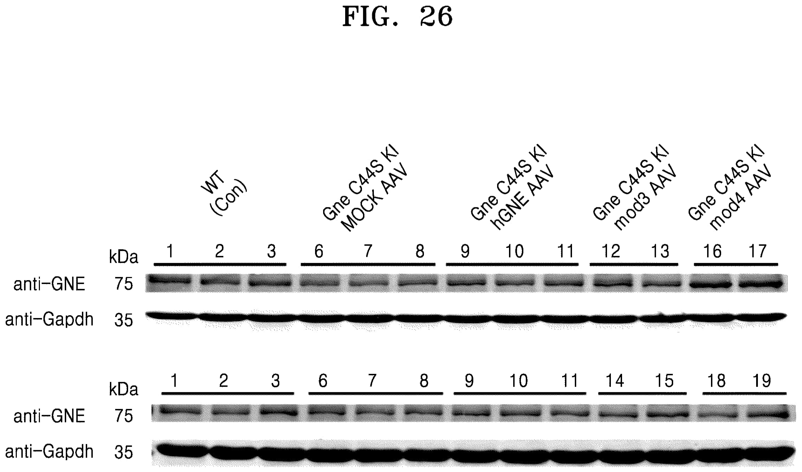

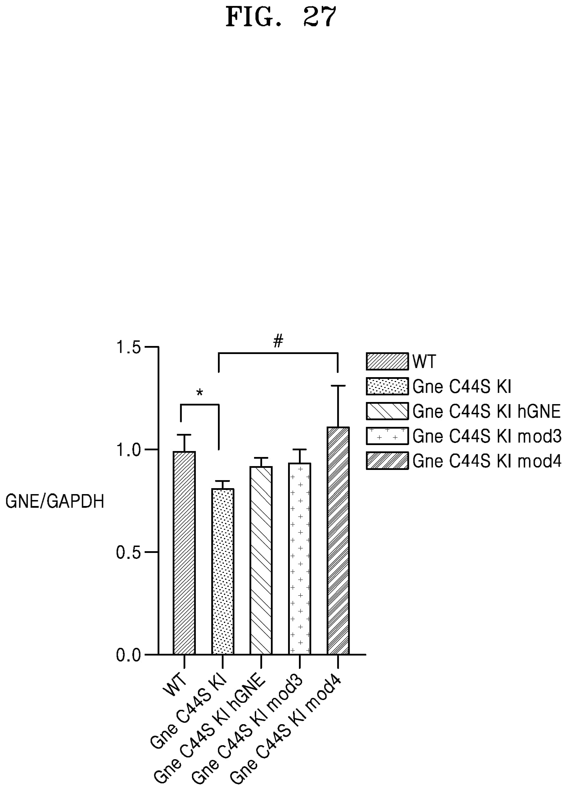

As a result, as shown in FIG. 26 and FIG. 27, in the group introduced with the recombinant AAV9 vector including the GNE nucleotide sequence according to an embodiment, the GNE expression level was restored and showed an expression level similar to control group or increased compared to the control group.

### Experimental Example 7. In Vivo Safety Evaluation Using Disease Mouse Model

In the present experimental example, the aim was to confirm the in vivo safety of the recombinant AAV9 vector by measuring the survival and changes in body weight of the disease mouse model of Experimental Example 6. Specifically, at 8 weeks post-administration of a recombinant AAV9 vector (Example 3 (Gne KI GNE mod3 AAV), Example 4 (Gne KI GNE mod3 AAV) including a GNE nucleotide sequence according to an embodiment of the disease mouse model, the survival of the subject was confirmed, and their body weights were measured a total of 4 times every 2 weeks during the period.

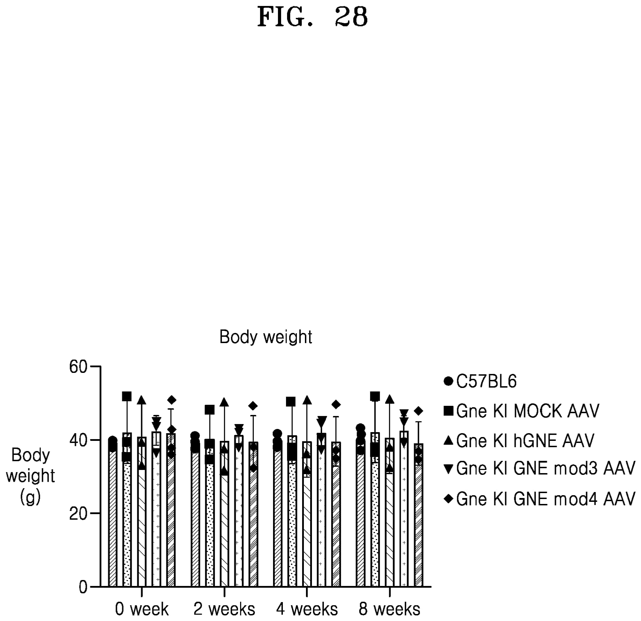

As a result, all groups introduced with a recombinant AAV9 vector including a GNE nucleotide sequence according to an embodiment survived, and as shown in FIG. 28, it was confirmed that there was no significant change in the body weight of the subjects during the experimental period.

### Experimental Example 8. Evaluation of Functional Recovery of Recombinant AAV9 Vector Including GNE Nucleotide Sequence of Example 3 Using Disease Mouse Model

In the present experimental example, the aim was to confirm the functional recovery by administration of a recombinant AAV9 vector including the GNE nucleotide sequence of Example 3. To this end, C44S knock-in mice over 40 weeks old were selected as a disease mouse model, and recombinant AAV9 vectors including the GNE nucleotide sequence of Example 3 at different administration doses, that is, Gne KI mod3 LD (low dose), Gne KI mod3 MD (Mid dose), and Gne KI mod3 HD (high dose), were administered to the disease mouse model. Thereafter, after 4 weeks post-administration, a treadmill test was conducted on subjects of each group. Along with the aforementioned behavioral evaluation, the body weight changes were measured in the disease mouse model to confirm the in vivo safety of the recombinant AAV9 vector. In addition, in the present experimental example, normal mice administered PBS (WT PBS) were used as a control group, and a group administered recombinant AAV9 with a wild-type GNE nucleotide sequence applied to a disease mouse model (Gne KI hGNE) was used as a comparison group. The mouse model and administration conditions in the present experimental example are as shown in Table 10 below.

**[Table 10]**

| **Group** | | **Age** | **Number** | **Dose (per 200** *µℓ***)** |
|---|---|---|---|---|
| **Normal** | **WT PBS** | 40-week-old or older (male) | 6 | PBS |
| **Gne C44S KI** | **hGNE AAV** | 40-week-old or older (male) | 6 | 3.3E+11 |
| | **LD mod3 AAV** | 40-week-old or older (male) | 6 | 3.3E+10 |
| | **MD mod3 AAV** | 40-week-old or older (male) | 6 | 1.0E+11 |
| | **HD mod3 AAV** | 40-week-old or older (male) | 7 | 3.3E+11 |

### 8-1. Behavioral Evaluation

A treadmill test was conducted according to the method of Experimental Example 6-1 on a disease mouse model administered with a recombinant AAV9 vector including a GNE nucleotide sequence according to an embodiment, to confirm the therapeutic efficacy of the recombinant AAV9 vector including the GNE nucleotide sequence of Example 3.

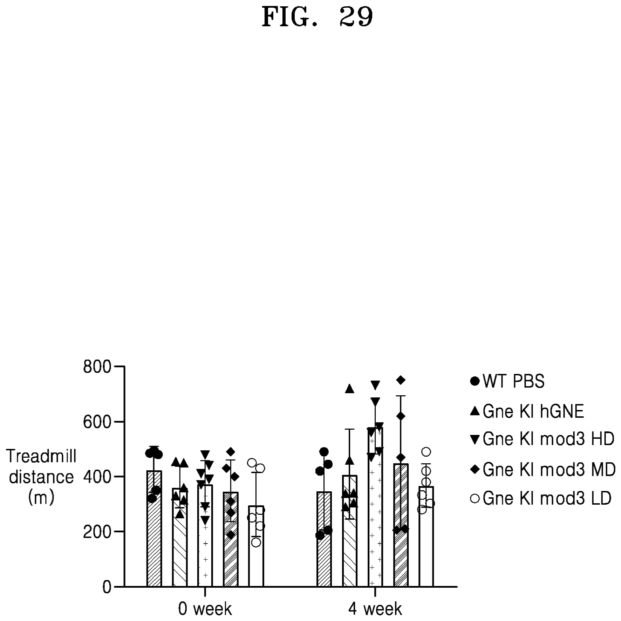

As a result, as shown in FIG. 29, it was confirmed that the travel distance of the treadmill was increased in the group introduced with the recombinant AAV9 vector including the GNE nucleotide sequence according to an embodiment, compared to the comparison group administered with the wild-type GNE nucleotide sequence.

### 8-2. In Vivo Safety Evaluation

The survival and body weight changes were measured in a disease mouse model administered with a recombinant AAV9 vector including a GNE nucleotide sequence according to an embodiment, to confirm the in vivo safety of the recombinant AAV9 vector including the GNE nucleotide sequence of Example 3. Specifically, at 4 weeks post-administration of a recombinant AAV9 vector including a GNE nucleotide sequence according to an embodiment, the survival of the subjects was confirmed and their body weights were measured.

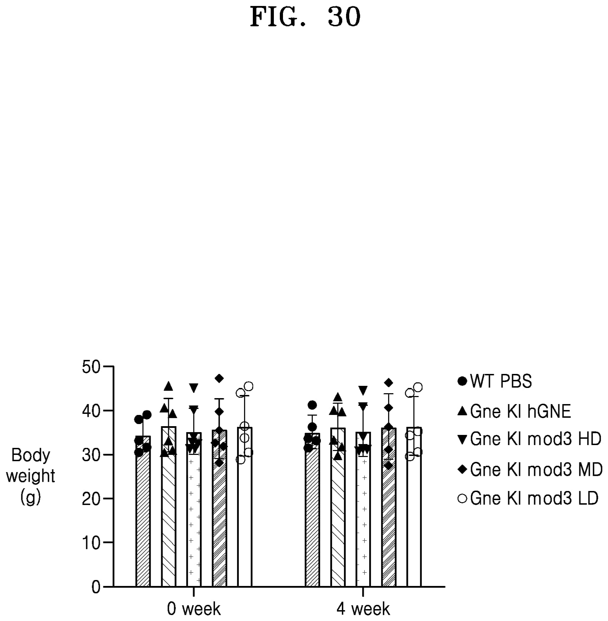

As a result, all groups introduced with a recombinant AAV9 vector including a GNE nucleotide sequence according to an embodiment survived, and as shown in FIG. 30, it was confirmed that there was no significant change in the body weight of the subjects during the experimental period.
The foregoing description of the present disclosure is for illustrative purposes only, and one that has ordinary skill in the art to which the present disclosure belongs will understand that the present disclosure may be readily adapted to other specific forms without altering the technical ideas or essential features of the present disclosure. Therefore, the examples described above should be understood in all respects as illustrative and not restrictive.

## Claims

1. An isolated nucleic acid satisfying the following conditions, wherein the isolated nucleic acid is any one selected from the group consisting of a nucleotide sequence of SEQ ID NO: 3, a nucleotide sequence of SEQ ID NO: 5, and a nucleotide sequence of SEQ ID NO: 6:
(a) encoding a GNE protein represented by an amino acid sequence of SEQ ID NO: 1;
(b) a sequence identity of 75 to 80 % with a nucleotide sequence of SEQ ID NO: 2; and
(c) a GC content of 55 to 58 % with respect to the entire nucleotide sequence.

2. An expression cassette comprising the isolated nucleic acid of claim 1.

3. The expression cassette of claim 2, comprising the following elements:
a first inverted terminal repeat (ITR);
a cytomegalovirus enhancer;
a cytomegalovirus promoter;
any one isolated nucleic acid selected from the group consisting of the nucleotide sequence of SEQ ID NO: 3, the nucleotide sequence of SEQ ID NO: 5, and the nucleotide sequence of SEQ ID NO: 6;
a human growth hormone gene 1 (hGH1) polyadenylation signal; and
a second inverted terminal repeat (ITR).

4. An adeno-associated virus serotype 9 (AAV9)-based recombinant virus, comprising the expression cassette of claim 2 or claim 3.

5. The recombinant virus of claim 4, wherein the recombinant virus enhances sialic acid production in muscle.

6. The recombinant virus of claim 4, wherein the recombinant virus increases an expression level of a GNE protein.

7. A pharmaceutical composition for preventing or treating GNE myopathy (GNEM), comprising as an active ingredient a vector comprising any one isolated nucleic acid selected from the group consisting of a nucleotide sequence of SEQ ID NO: 3, a nucleotide sequence of SEQ ID NO: 5, and a nucleotide sequence of SEQ ID NO: 6.

8. The pharmaceutical composition of claim 7, wherein the vector is an adeno-associated virus serotype 9 (AAV9)-based recombinant virus.
